# EUROPEAN PATENT APPLICATION

(11) **EP 4 483 919 A1**
(43) Date of publication of application: **01.01.2025**
(21) Application number: 24185200.3
(22) Date of filing: 28.06.2024
(51) Int. Cl.: A61M 1/36, B04B 5/04, B04B 3/00

(54) **SYSTEM AND METHOD FOR PROCESSING OF WHOLE BLOOD INTO PLASMA PRODUCTS OF DIFFERENT CATEGORIZATION**

(30) Priority: 30.06.2023 US 202363511377 P; 27.07.2023 US 202363529233 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US); MADSEN, James G., Lake Zurich, 60047 (US)
(74) Representative: Maikowski & Ninnemann Patentanwälte Partnerschaft mbB

(57) **Abstract**

A blood processing device includes a pump system, a valve system, a separator and a controller configured to execute a plasma collection stage of a blood processing procedure by actuating the pump system and the valve system to convey blood from a blood source into the separator, actuating the separator to separate the blood into at least plasma and red blood cells, in a transfusion plasma collection step actuating the pump system and the valve system to convey a first volume of separated plasma out of the separator along a first flow path for transfusion plasma collection, and in a fractionation plasma collection step of the plasma collection stage, actuating the pump system and valve system to convey a second volume of the separated plasma out of the separator along a second flow path for fractionation plasma collection.

## Description

### Background

### Field of the Disclosure

The present disclosure relates to separation of whole blood and collection of separated blood components. More particularly, the present disclosure relates to the collection of plasma products in different product categories.

### Description of Related Art

Various blood processing systems make it possible to separate whole blood into particular blood constituents and collect the separated blood constituents as various blood products for later use. Typically, in such systems, whole blood is drawn from a blood source, such as a whole blood bag or a human donor, and the particular blood component or constituent is separated, removed, and collected. In the case of a human donor, the remaining blood constituents may be returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

According to one approach, whole blood may be separated into its constituents through centrifugation. This requires that the whole blood be passed through a centrifuge after it is drawn from the blood source. To reduce contamination and possible infection (if the blood source is a human donor or patient), the blood is preferably processed within a sealed, sterile fluid flow circuit during the centrifugation process. To this end, the operator installs a fresh, sterile disposable flow circuit in the centrifuge before processing and removes and discards the flow circuit afterwards. Typical disposable flow circuits are sealed and sterile, and include a separation chamber portion, which is mounted in cooperation on a durable, reusable assembly containing the hardware (centrifuge, drive system, pumps, valve actuators, programmable controller, and the like) that rotates the separation chamber and controls the flow through the fluid circuit. The separation chamber may be formed of a generally rigid material (e.g., molded plastic), in which case the chamber itself defines a flow path or channel in which blood is separated into two or more components, or a more flexible material (e.g., in the form of a belt or annulus), which relies upon the system hardware to support the chamber and define the shape of the chamber as blood flows through it.

With a disposable circuit loaded onto the centrifuge (or just prior to or during loading) the operator typically enters, for example, by means of a touch screen or other user interface system, a particular processing protocol to be executed by the system (e.g., a procedure wherein platelets are separated from whole blood and collected) and other parameters (e.g., the weight of the donor, the desired volume of separated blood component to be collected, etc.). Whole blood is provided to the disposable circuit by connecting the whole blood bag to the circuit, or in the case of a human donor, by phlebotomizing a donor (or otherwise accesses the blood of the blood source). The system, once programmed, carries out the procedure under the supervision of the operator.

The centrifuge rotates the separation chamber of the disposable flow circuit during processing, causing the heavier (greater specific gravity) components of the whole blood in the separation chamber, such as red blood cells, to move radially outwardly away from the center of rotation toward the outer or "high-G" wall of the separation chamber. The lighter (lower specific gravity) components, such as plasma, migrate toward the inner or "low-G" wall of the separation chamber. The boundary that forms between the heavier and lighter components in the separation chamber is commonly referred to as the interface. Various ones of these components can be selectively removed from the whole blood by providing appropriately located channeling structures and outlet ports in the flow circuit. For example, in one blood separation procedure, blood is separated into plasma and red blood cells, with a buffy coat or white blood cell layer positioned therebetween. The buffy coat or white blood cell layer (which may include platelets and various white blood cells, such as mononuclear cells and possibly peripheral blood stem cells) is collected, with plasma and red blood cells being returned to the blood source or separately collected.

A system capable of executing such a red blood cell, plasma, and buffy coat collection procedure is described in PCT Patent Application Publication No. WO 2021/194824 A1, which is hereby incorporated herein by reference. More particularly, PCT Patent Application Publication No. WO 2021/194824 A1 describes a procedure in which blood in a continuous-flow centrifuge chamber is separated into plasma, buffy coat, and red blood cells. Separated plasma exits the chamber via a plasma outlet port and is collected in a plasma collection container, while separated red blood cells exit the chamber via a red blood cell outlet port and are collected in a red blood cell collection container. The buffy coat remains within the chamber while the other components are being collected.

Plasma collected in manual whole blood processing and apheresis procedures is categorized as either a "transfusion" plasma product intended to be transfused or a "fractionation" plasma product intended for fractionation. Transfusion plasma is sold per unit and typically requires a minimum volume of 180 mL, with greater volumes not offering any additional value for a blood center selling the product. Fractionation plasma is sold per unit volume (e.g., per mL). Thus, the total volume of fractionation plasma product will determine the product's value.

In current whole blood manufacturing processes, a predetermined number of transfusion plasma products per day are sent to hospitals or other medical facilities for transfusion based on needs while remaining plasma products are sold to fractionation, which can be a significant profit driver for blood manufacturing centers.

However, current disposable circuit configurations and separation processes do not enable collection of plasma from a single whole blood unit into multiple plasma product bags. Thus, using the current circuit configurations and separation processes may result in transfusion plasma product units having a plasma volume exceeding 180 mL, which as indicated above, does not offer additional value over a transfusion plasma product unit containing only 180 mL of plasma.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood processing device includes a pump system, a valve system, a separator and a controller operably connected to the pump system, the valve system and the separator, the controller configured to execute a plasma collection stage of a blood processing procedure by: actuating the pump system and the valve system to convey blood from a blood source into the separator, actuating the separator to separate the blood in the centrifuge into at least plasma and red blood cells, in a transfusion plasma collection step of the plasma collection stage, actuating the pump system and the valve system to convey a first volume of the separated plasma out of the separator for collection, wherein the valve system is actuated to convey the first volume of the separated plasma along a first flow path for transfusion plasma collection, and in a fractionation plasma collection step of the plasma collection stage, actuating the pump system and valve system to convey a second volume of the separated plasma out of the centrifuge for collection, wherein the valve system is actuated to convey the second volume of the plasma along a second flow path for fractionation plasma collection.

In another aspect, a blood processing system includes a reusable processing device including a pump system, a valve system a separator and a controller, and a disposable fluid flow circuit including a processing chamber interacting with the separator, a blood supply container, a transfusion plasma collection container, a fractionation plasma collection container and a red blood cell collection container, and a plurality of conduits connecting the processing chamber, the blood supply container, the transfusion plasma collection container, the fractionation plasma collection container and the red blood cell collection container. The controller is configured to execute a plasma collection stage by: actuating the pump system and the valve system to convey blood from the blood supply container into the processing chamber, actuating the separator to separate the blood in the processing chamber into plasma and red blood cells, in a transfusion plasma collection step of the plasma collection stage, actuating the pump system and the valve system to convey a first volume of the separated plasma from the processing chamber via a first outlet for collection in the transfusion plasma collection container, wherein the valve system is actuated to convey the first volume of the separated plasma along a first flow path to the transfusion plasma collection container, in a fractionation plasma collection step, actuating the pump system and the valve system to convey a second volume of the separated plasma from the processing chamber via the first outlet for collection in the fractionation plasma collection container, wherein the valve system is actuated to convey the second volume of the separated plasma along a second flow path to the fractionation plasma collection container, and actuating the pump system and the valve system to convey the separated red blood cells from the processing chamber via a second outlet for collection in the red blood cell container.

In yet another aspect, a fluid separation device includes a pump system, a centrifuge, and a controller. The centrifuge is configured to receive a separation chamber comprising an inlet port and an outlet port and to rotate the separation chamber about a non-vertical, non-horizontal rotational axis. The controller is programmed to execute a fluid separation procedure including a "priming" stage and/or an "air harvest" stage. During the "priming" stage, the controller controls the centrifuge to rotate the separation chamber to a first target orientation and then controls the pump system to convey a priming fluid into the separation chamber via the inlet port to remove air from the separation chamber via the outlet port while retaining the separation chamber in the first target orientation. During the "air harvest" stage, the controller controls the centrifuge to rotate the separation chamber to a second target orientation and then controls the pump system to convey air into the separation chamber via one of the inlet port and the outlet port to remove fluid and/or a fluid component from the separation chamber via the other one of the inlet port and the outlet port while retaining the separation chamber in the second target orientation.

In another aspect, a method is provided for priming a separation chamber received within a centrifuge. The method includes controlling the centrifuge to rotate the separation chamber about a non-vertical, non-horizontal rotational axis to a target orientation and then controlling a pump system to convey a priming fluid into the separation chamber via an inlet port to remove air from the separation chamber via an outlet port while retaining the separation chamber in the target orientation.

In yet another aspect, a method is provided for harvesting a fluid and/or a fluid component from a separation chamber received within a centrifuge. The method includes controlling the centrifuge to rotate the separation chamber about a non-vertical, non-horizontal rotational axis to a target orientation and then controlling a pump system to convey air into the separation chamber via a first port to remove a fluid and/or a fluid component from the separation chamber via a second port while retaining the separation chamber in the target orientation.

### Brief Description of the Drawings

Fig. 1 is a perspective view of an exemplary reusable hardware component of a blood processing system which is configured to receive a disposable fluid flow circuit;
Fig. 2 is a plan view of an exemplary disposable fluid flow circuit for use in combination with the durable hardware component of Fig. 1;
Fig. 3 is a schematic view of the fluid flow circuit of Fig. 2 mounted to the processing device of Fig. 1 to complete a blood processing system according to an aspect of the present disclosure;
Fig. 4 is a schematic view of the blood processing system of Fig. 3 executing a "blood prime" stage of an exemplary blood processing procedure;
Fig. 5 is a schematic view of the blood processing system of Fig. 3 executing an "establish separation" stage of an exemplary blood processing procedure;
Fig. 6 is a schematic view of the blood processing system of Fig. 3 executing a transfusion plasma collection step of a "plasma collection" stage of an exemplary blood processing procedure, in which separated red blood cells are directed through a leukoreduction filter;
Fig. 7 is a schematic view of the blood processing system of Fig. 3 executing a transfusion plasma collection step of a "plasma collection" stage of an exemplary blood processing procedure, in which separated red blood cells bypass a leukoreduction filter;
Fig. 8 is a schematic view of the blood processing system of Fig. 3 executing a fractionation plasma collection step of a "plasma collection" stage of an exemplary blood processing procedure, in which separated red blood cells are directed through a leukoreduction filter;
Fig. 9 is a schematic view of the blood processing system of Fig. 3 executing a fractionation plasma collection step of a "plasma collection" stage of an exemplary blood processing procedure, in which separated red blood cells bypass a leukoreduction filter;
Fig. 10 is a schematic view of the blood processing system of Fig. 3 executing a "red blood cell recovery" stage of an exemplary blood processing procedure, in which separated red blood cells are directed through a leukoreduction filter;
Fig. 11 is a schematic view of the blood processing system of Fig. 3 executing a "red blood cell recovery" stage of an exemplary blood processing procedure, in which separated red blood cells bypass a leukoreduction filter;
Fig. 12 is a schematic view of the blood processing system of Fig. 3 executing an "additive solution flush" stage of an exemplary blood processing procedure, in which additive solution is directed through a leukoreduction filter;
Fig. 13 is a schematic view of the blood processing system of Fig. 3 executing an "additive solution flush" stage of an exemplary blood processing procedure, in which additive solution bypasses a leukoreduction filter;
Fig. 14 is a schematic view of the blood processing system of Fig. 3 executing an "air evacuation" stage of an exemplary blood processing procedure;
Fig. 15 is a front elevational view of a centrifuge of the reusable hardware component of Fig. 1, with a fluid separation chamber mounted thereto;
Fig. 16 is a front elevational view of the centrifuge and fluid separation chamber of Fig. 15, oriented at an angle θ, according to an aspect of the present disclosure;
Fig. 17 is a detailed view of the fluid separation chamber of Fig. 16, oriented so as to place outlet ports of the fluid separation chamber at a "high point;"
Fig. 18 is a perspective view of fluid flow through the fluid separation chamber of Fig. 16, when in the orientation of Fig. 17;
Fig. 19 is a detailed view of the fluid separation chamber of Fig. 16, oriented so as to place an inlet port of the fluid separation chamber at a "low point;" and
Fig. 20 is a perspective view of fluid flow through the fluid separation chamber of Fig. 16, when in the orientation of Fig. 19.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

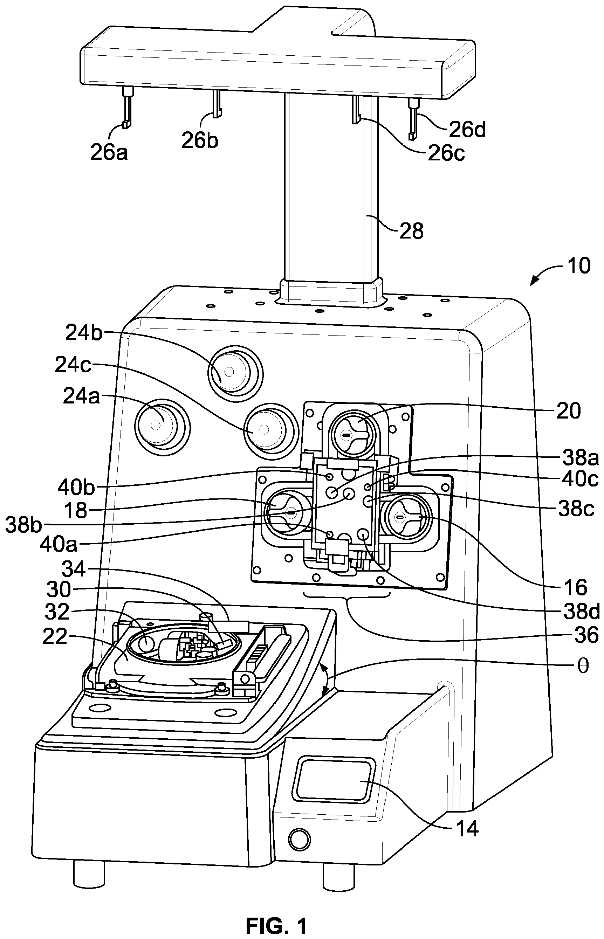

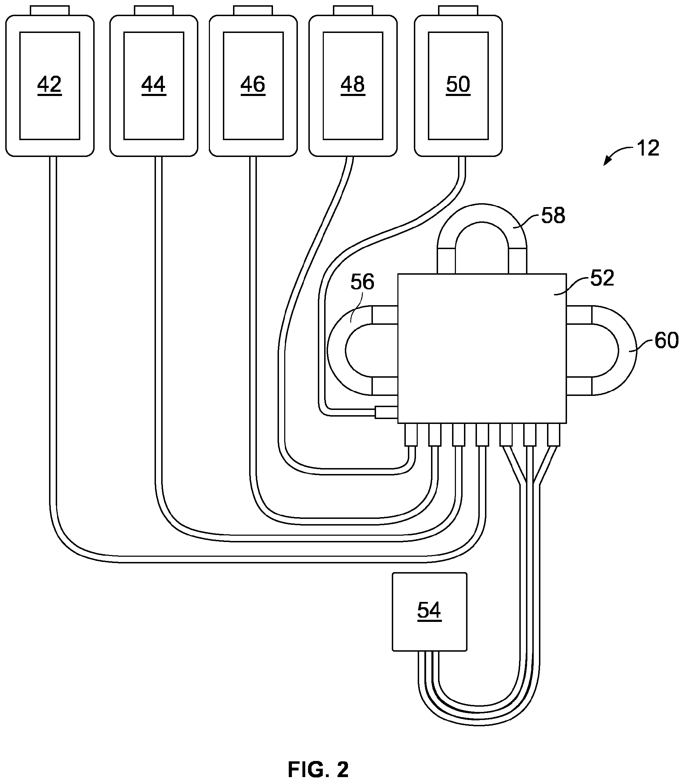

Fig. 1 depicts a reusable hardware component or fluid separation or processing device of a blood processing system, generally designated 10, while Fig. 2 depicts a disposable fluid flow circuit, generally designated 12, to be used in combination with the processing device 10 for processing blood. The illustrated processing device 10 includes associated pumps, valves, sensors, displays, and other apparatus for configuring and controlling flow of fluid through the fluid flow circuit 12, described in greater detail below. The blood processing system may be directed by a controller integral with the processing device 10 that includes a programmable microprocessor to automatically control the operation of the pumps, valves, sensors, etc. The processing device 10 may also include wireless communication capabilities to enable the transfer of data from the processing device 10 to the quality management systems of the operator.

More specifically, the illustrated processing device 10 includes a user input and output touchscreen 14, a pump station including a first pump 16 (for pumping, e.g., whole blood), a second pump 18 (for pumping, e.g., plasma) and a third pump 20 (for pumping, e.g., additive solution), a separator which may be provided as a centrifuge mounting station and drive unit 22 (which may be referred to herein as a "centrifuge"), and clamps 24a-c. The touchscreen 14 enables user interaction with the processing device 10, as well as the monitoring of procedure parameters, such as flow rates, container weights, pressures, etc. The pumps 16, 18, and 20 (collectively referred to herein as being part of a "pump system" of the processing device 10) are illustrated as peristaltic pumps capable of receiving tubing or conduits and moving fluid at various rates through the associated conduit dependent upon the procedure being performed. An exemplary centrifuge mounting station/drive unit is seen in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits and may incorporate RF sealers in order to complete a heat seal of the tubing or conduit placed in the clamp to seal the tubing or conduit leading to a product container upon completion of a procedure.

Sterile connection/docking devices may also be incorporated into one or more of the clamps 24a-c. The sterile connection devices may employ any of several different operating principles. For example, known sterile connection devices and systems include radiant energy systems that melt facing membranes of fluid flow conduits, as in U.S. Patent No. 4,157,723; heated wafer systems that employ wafers for cutting and heat bonding or splicing tubing segments together while the ends remain molten or semi-molten, such as in U.S. Patent Nos. 4,753,697; 5,158,630; and 5,156,701; and systems employing removable closure films or webs sealed to the ends of tubing segments as described, for example, in U.S. Patent No. 10,307,582. Alternatively, sterile connections may be formed by compressing or pinching a sealed tubing segment, heating and severing the sealed end, and joining the tubing to a similarly treated tubing segment as in, for example, U.S. Patent Nos. 10,040,247 and 9,440,396. All of the above-identified patents are incorporated by reference in their entirety. Sterile connection devices based on other operating principles may also be employed without departing from the scope of the present disclosure.

The processing device 10 also includes hangers 26a-d (which may each be associated with a weight scale or sensor) for suspending the various containers of the disposable fluid circuit 12. The hangers 26a-d are preferably mounted to a support 28, which is vertically translatable to improve the transportability of the processing device 10. An optical system comprising a laser 30 and a photodetector 32 is associated with the centrifuge 22 for determining and controlling the location of an interface between separated blood components within the centrifuge 22. An exemplary optical system is shown in U.S. Patent Application Publication No. 2019/0201916, which is hereby incorporated herein by reference. An optical sensor 34 is also provided to optically monitor one or more conduits leading into or out of the centrifuge 22. The example illustrated in Fig. 1 shows four hangers 26a-d. However, it will be appreciated that additional or fewer hangers may be provided to support the various containers of the disposable fluid circuit 12. For example, the processing device 10 may be provided with five hangers to support up to five containers of a selected disposable fluid circuit 12.

The face of the processing device 10 includes a nesting module 36 for seating a flow control cassette 52 (Fig. 2) of the fluid flow circuit 12. The cassette nesting module 36 is configured to receive various disposable cassette designs so that the system may be used to perform different types of procedures. Embedded within the illustrated cassette nesting module 36 are four valves 38a-d (collectively referred to herein as being part of the "valve system" of the processing device 10) for opening and closing fluid flow paths within the flow control cassette 52, and three pressure sensors 40a-c capable of measuring the pressure at various locations of the fluid flow circuit 12.

With reference to Fig. 2, the illustrated fluid flow circuit 12 includes a plurality of containers 42, 44, 46, 48, and 50 with a flow control cassette 52 and a processing/separation chamber 54 that is configured to be received in the centrifuge 22, all of which are interconnected by conduits or tubing segments, so as to permit continuous flow centrifugation. The flow control cassette 52 routes the fluid flow through three tubing loops 56, 58, 60, with each loop being positioned to engage a particular one of the pumps 16, 18, 20. The conduits or tubing may extend through the cassette 52, or the cassette 52 may have pre-formed fluid flow paths that direct the fluid flow.

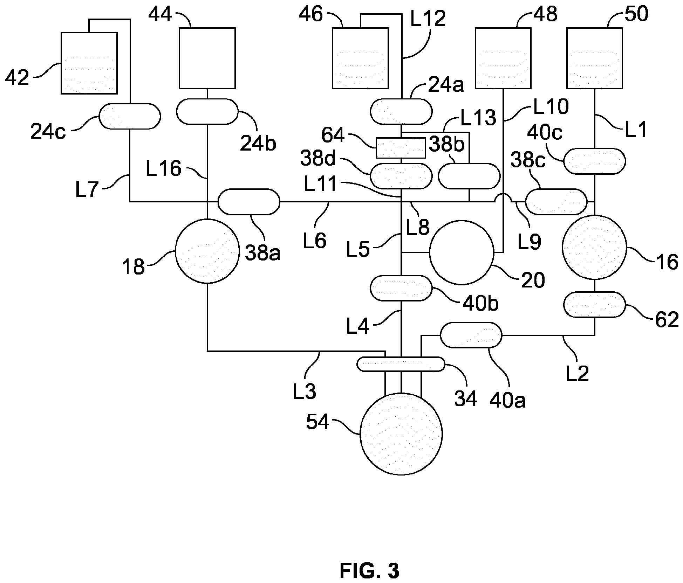

In the fluid flow circuit 12 shown in Fig. 2, containers 42, 44, and 46 may be empty containers for the receipt of transfusion plasma, fractionation plasma and red blood cells separated from whole blood as described further below, container 48 may be pre-filled with additive solution, and container 50 may be filled with whole blood and connected to the fluid flow circuit 12 at the time of use. While Fig. 2 shows a whole blood container 50 (configured as a blood pack unit, for example) as a blood source, it is within the scope of the present disclosure for the blood source to be a living donor. The fluid flow circuit 12 may optionally include an air trap 62 (Fig. 3) through which the whole blood is flowed prior to entering the separation chamber and/or an optional leukoreduction filter 64 through which the red blood cells may be flowed prior to entering the red blood cell collection container 46.

The processing chamber 54 may be pre-formed in a desired shape and configuration by injection molding from a rigid plastic material, as shown and described in U.S. Patent No. 6,849,039, which is hereby incorporated herein by reference. The specific geometry of the processing chamber 54 may vary depending on the elements to be separated, and the present disclosure is not limited to the use of any specific chamber design. For example, it is within the scope of the present disclosure for the processing chamber 54 to be configured formed of a generally flexible material, rather than a generally rigid material. When the processing chamber 54 is formed of a generally flexible material, it relies upon the centrifuge 22 to define a shape of the processing chamber 54. An exemplary processing chamber formed of a flexible material and an associated centrifuge are described in U.S. Patent No. 6,899,666, which is hereby incorporated herein by reference.

The controller of the processing device 10 may be pre-programmed to automatically operate the system to perform one or more standard blood processing procedures selected by an operator by input to the touchscreen 14, and configured to be further programmed by the operator to perform additional blood processing procedures. The controller may be pre-programmed to substantially automate a wide variety of procedures, including, but not limited to: red blood cell and plasma production from a single unit of whole blood, buffy coat pooling, buffy coat separation into a platelet product (as described in U.S. Patent Application Publication No. 2018/0078582, which is hereby incorporated herein by reference), glycerol addition to red blood cells, red blood cell washing, platelet washing, and cryoprecipitate pooling and separation.

The pre-programmed blood processing procedures operate the system at pre-set settings for flow rates and centrifugation forces, and the programmable controller may be further configured to receive input from the operator as to one or more of flow rates and centrifugation forces for the standard blood processing procedure to override the pre-programmed settings. In addition, the programmable controller may be configured to receive input from the operator through the touchscreen 14 for operating the system to perform a non-standard blood processing procedure.

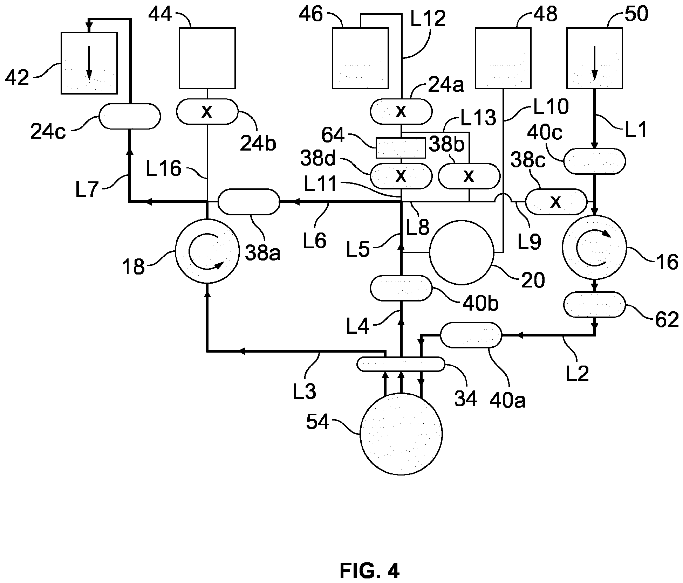

According to an aspect of the present disclosure, the processing device 10 and the fluid flow circuit 12 may be used in combination to process a volume of whole blood into a transfusion plasma product, a fractionation plasma product and optionally a red blood cell product. Fig. 3 is a schematic illustration of the fluid flow circuit 12 mounted to the processing device 10, with selected components of the fluid flow circuit 12 and selected components of the processing device 10 being shown. Figs. 4-14 show different stages of an exemplary procedure. In the procedure to be described, plasma is separated from cellular blood components as platelet-poor plasma, with platelets separated from the plasma settling into the buffy coat layer (along with assorted white blood cells). In other embodiments, a separated plasma layer may comprise platelet-rich plasma, in which case there is an intermediate layer of white blood cells (which may be predominantly mononuclear cells and peripheral blood stem cells) between the plasma layer and the red blood cell layer. In either case, the procedure is similar, such that references herein to "plasma" or a "plasma layer" are intended to encompass both platelet-poor plasma and platelet-rich plasma, while the intermediate blood component layer (between the plasma layer and the red blood cell layer) will be referred to as the buffy coat or white blood cell layer (with it being understood that a buffy coat accompanies a platelet-poor plasma layer while a white blood cell layer accompanies a platelet-rich plasma layer).

### "Blood Prime" Stage

In an initial stage, shown in Fig. 4, selected components of the fluid flow circuit 12 are primed to remove air therefrom in a "blood prime" or "priming" stage. While it is in the scope of the present disclosure for a separately provided fluid (e.g., anticoagulant or saline) to be used for priming the fluid flow circuit 12, the Fig. 4 illustrates a blood prime stage in which regions of the fluid flow circuit 12 are primed using whole blood from a blood source. The blood source may be the whole blood container 50 as shown in Fig. 4 or may alternatively be a living donor. Thus, it should be understood that the term "whole blood" may refer to blood that either includes or omits an anticoagulant fluid. It will be further appreciated that the following examples may refer to the "whole blood container 50" or "blood supply container 50" to use terminology consistent with the examples depicted in Figs. 2-14, but that such terminology does not preclude a living donor as the blood source in other examples.

During the blood prime stage, whole blood is drawn into the fluid flow circuit from the whole blood container 50 via line L1 by operation of the first pump 16 (which may be referred to as the "whole blood pump"). Valve 38c is closed, which directs the blood through pressure sensor 40c and into line L2. The blood passes through air trap 62, pressure sensor 40a (which measures the pressure of processing chamber 54), and optical sensor 34 before flowing into the processing chamber 54, which is positioned within the separator, which may be provided as centrifuge 22, of the processing device 10. It will be appreciated that the centrifuge 22 may be referenced throughout the following examples in order to use consistent terminology across the various examples. However, the present examples are not limited to a centrifuge 22 of the types described below, and other separators are within the scope of this disclosure.

The centrifuge 22 may be stationary during the blood prime stage or may instead be controlled by the controller of the processing device 10 to spin at a low rotation rate (e.g., on the order of approximately 1,000-2,000 rpm) such that the blood remains unseparated in the processing chamber 54. It may be advantageous for the centrifuge 22 to rotate during the blood prime stage in order to create enough g-force to ensure that the air in the processing chamber 54 (which includes air already present in the processing chamber 54, along with air moved into the processing chamber 54 from lines L1 and/or L2 by the flow of blood) is forced towards the low-G (radially inner) wall of the processing chamber 54. Higher centrifuge rotation rates, such as 4,500 rpm (which is required for steady state separation, as will be described) may be undesirable as air blocks (in which air gets stuck and cannot be forced out of the processing chamber 54, causing pressure to rise) are more likely at higher g-forces.

The blood entering the processing chamber 54 will move towards the high-g (radially outer) wall of the processing chamber 54, displacing air towards the low-g wall. A plasma outlet port, also referred to herein as a first outlet, of the processing chamber 54 is associated with the low-G wall of the processing chamber 54, such that most of the air will exit the processing chamber 54 via the plasma outlet port and associated line L3, although some air may also exit processing chamber 54 via a red blood cell outlet port, also referred to herein as a second outlet, associated with the high-G wall of the processing chamber 54.

Valves 38b and 38d and clamp 24b are closed, while the second pump 18 (which may be referred to as the "plasma pump") is active and the third pump 20 (which may be referred to as the "additive pump") is inactive. Such an arrangement will direct the air exiting the processing chamber 54 via the red blood cell outlet port through associated line L4 and pressure sensor 40b, into line L5 and then into line L6. Valve 38a is open, such that the air flowing through line L6 will meet up with the air flowing through line L3 (i.e., the air that exits the processing chamber 54 via the plasma outlet port). The combined air will flow through line L7 and open clamp 24c, into the transfusion plasma collection container 42.

It should be understood that, in Figs. 4-14, arrows on the containers represent the direction of fluid flow between the container and the conduit connected to the container. For example, line L7 is shown as being connected to the top of the transfusion plasma collection container 42, such that a downward arrow (as in Fig. 4) represents downward fluid flow into the transfusion plasma collection container 42. In contrast, line L1 is shown as being connected to the bottom of the whole blood container 50, such that a downward arrow (as in Fig. 4) represents downward fluid flow out of the whole blood container 50.

The flow of air out of the processing chamber 54 via either outlet port is monitored by the optical sensor 34, which is capable of determining the optical density of the fluid flowing through the monitored lines and discerning between air and a non-air fluid in lines L3 and L4. When a non-air fluid is detected in both lines L3 and L4, the controller of the processing device 10 will end the blood prime stage and move on to the next stage of the procedure. The amount of blood drawn into the fluid flow circuit 12 from the whole blood container 50 during the blood prime stage will vary depending on a number of factors (e.g., the amount of air in the fluid flow circuit 12), but may be on the order of approximately 50 to 100 mL. The blood prime stage may take on the order of one to two minutes.

Thus, in the blood prime stage, the controller may actuate the pump system and valve system to convey blood from the whole blood container 50 into the processing chamber 54 and actuate the centrifuge at a first rotation rate such that the blood remains unseparated. The controller may then actuate the pump system and valve system to convey a first portion of the unseparated blood from the processing chamber 54 via the first outlet to the transfusion plasma collection container 42 along a flow path including lines L3 and L7, which may be referred to herein as a first flow path. The controller may also actuate the pump system and the valve system to convey a second portion of the unseparated blood from the processing chamber 54 to the transfusion plasma collection container 42 along a flow path including lines L4, L5, L6 and L7, which may be referred to herein as a third flow path.

### "Establish Separation" Stage

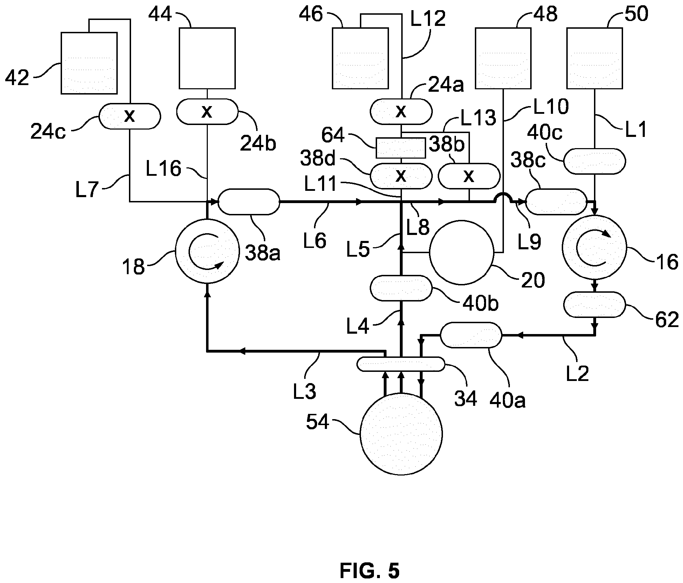

Fig. 5 shows an optional stage referred to herein as the "establish separation" stage, which may follow the blood prime stage illustrated in Fig. 4. When the supply of blood is limited and the system must work with a finite fluid volume, the establish separation stage is advantageous to avoid product loss or quality issues. These goals are achieved by separated plasma and red blood cells being removed from the processing chamber 54, mixed together to form recombined or reconstituted whole blood, and recirculated back into the processing chamber 54, rather than the separated components being directed to their respective collection containers, as described below.

The establish separation stage may be executed when non-air fluid has been detected in lines L3 and L4. In the establish separation stage, the rotational speed of the centrifuge 22 is increased to a rate that is sufficient to separate blood into packed red blood cells and platelet-poor plasma, for example, in the range of approximately 4,500 to 5,500 rpm. The whole blood pump 16 continues operating, but without drawing additional blood into the fluid flow circuit from the blood source 50 as described below.

If it is desired to produce a plasma product that is low in platelets, the processing chamber 54 may be configured with a plasma outlet port, i.e., a first outlet, that is spaced from and positioned downstream of the blood inlet port, rather than being positioned adjacent to the blood inlet port. Such a configuration allows the platelets to settle down into the buffy coat before the plasma is removed from the processing chamber 54, thus allowing the separated plasma to be platelet-depleted. A differently configured processing chamber 54 (and a different, lower rotational speed) may be employed if a platelet-rich plasma layer is to be separated from the blood.

Separated plasma will exit the processing chamber 54 via the plasma outlet port and associated line L3. Clamps 24b and 24c are closed during this stage, while valve 38a remains open, which directs the plasma from line L3 into line L6. Separated red blood cells exit the processing chamber 54 via the red blood cell outlet port, i.e., the second outlet, and associated line L4. In the illustrated example, there is no pump associated with line L4, such that the red blood cells exit the processing chamber 54 at a rate that is equal to the difference between the rate of the whole blood pump 16 and the rate of the plasma pump 18. However, in alternative embodiments, there may be a pump associated with the red blood cell outlet line (i.e., line L4) instead of the plasma outlet line (i.e., line L3) or a first pump associated with the plasma outlet line (i.e., line L3) and a second pump associated with the red blood cell outlet line (i.e., line L4).

The additive pump 20 is inactive during this stage, thereby directing the red blood cells from line L4 into line L5. The plasma flowing through line L6 is mixed with the red blood cells flowing through line L5 at the junction of the two lines L5 and L6 to form recombined whole blood. Valve 38d is closed, which directs the recombined whole blood into line L8. Valve 38b is also closed, which directs the recombined whole blood from line L8 into line L9 and through open valve 38c. The whole blood pump 16 draws the recombined whole blood into line L2 from line L9 (rather than drawing additional blood into the fluid flow circuit 12 from the whole blood container 50), with the recombined blood passing through air trap 62, pressure sensor 40a, and optical sensor 34 before flowing back into the processing chamber 54, where it is again separated into plasma and red blood cells, with buffy coat therebetween.

The establish separation stage continues until steady state separation has been achieved, which may take on the order of approximately one to two minutes. As used herein, the phrase "steady state separation" refers to a state in which blood is separated into its constituents in the processing chamber 54, with the radial position of the interface between separated components within the processing chamber 54 being at least substantially maintained (rather than moving radially inwardly or outwardly). The position of the interface may be determined and controlled according to any suitable approach, including using an interface detector of the type described in U.S. Patent Application Publication No. 2019/0201916.

Preferably, steady state separation is achieved with the interface between separated components within the processing chamber 54 at a target location. The target location may correspond to the location of the interface at which separation efficiency is optimized, with the precise location varying depending on a number of factors (e.g., the hematocrit of the whole blood). However, in an exemplary embodiment, the target location of the interface may be the position of the interface when approximately 52% of the thickness or width (in a radial direction) of the channel defined by the processing chamber 54 is occupied by red blood cells. In the illustrated embodiment, the position of the interface within the processing chamber 54 may be adjusted by changing the flow rate of the plasma pump 18, with the flow rate being increased to draw more separated plasma out of the processing chamber 54 (which decreases the thickness of the plasma layer within the processing chamber 54) and move the interface toward the low-G wall or decreased to draw less plasma out of the processing chamber 54 (which increases the thickness of the plasma layer within the processing chamber 54) and move the interface toward the high-G wall.

Thus, in the establish separation stage, the controller may actuate the pump system and the valve system to convey blood from the whole blood container 50 into the processing chamber 54 and actuate the centrifuge at a second rotation rate to separate the whole blood into at least plasma and red blood cells. The pump system and the valve system may also be actuated to convey the separated plasma from the processing chamber 54 via the first (plasma) outlet and to convey the separated red blood cells from the processing chamber 54 via the second (red blood cell) outlet. The separated plasma is conveyed along a flow path including lines L3 and L6, and the red blood cells are conveyed along a flow path including lines L4 and L5. The pump system and valve system are also actuated to combine the separated plasma and the separated red blood cells as reconstituted blood in line L8 and convey the reconstituted blood back to the processing chamber 54 along a flow path including lines L9 and L2.

### "Plasma Collection" Stage

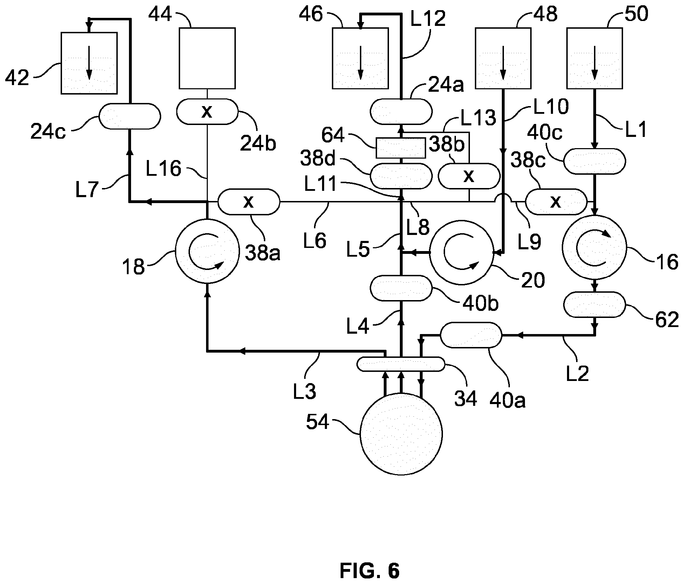

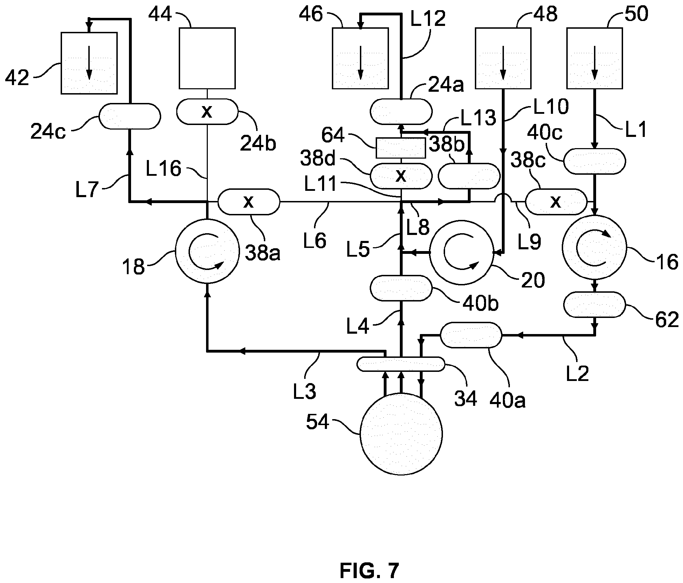

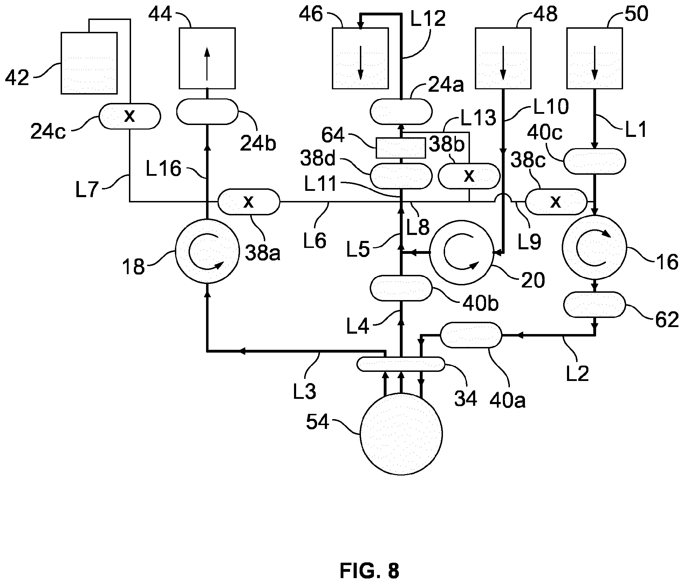

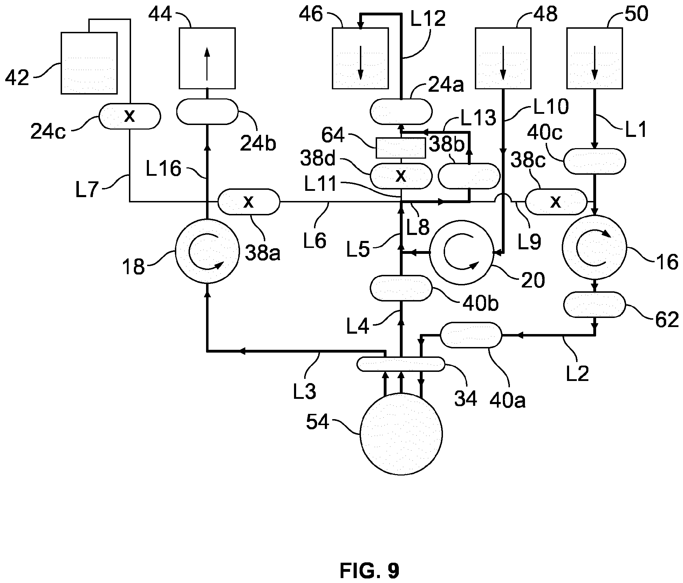

With reference to Figs. 6-9, once steady state separation has been achieved, the controller ends the establish separation stage and advances the procedure to a "plasma collection" stage. In the present examples, the plasma collection stage includes a transfusion plasma collection step (Figs. 6 and 7) in which a first volume of plasma is collected in the transfusion plasma collection container 42 to provide a transfusion plasma product and a fractionation plasma collection step (Figs. 8 and 9) in which a second volume of plasma is collected in the fractionation plasma collection container 44 to provide a fractionation plasma product. The controller may control the processing device 10 to switch from the transfusion plasma collection step to the fractionation plasma collection step, for example, in response to determining the first volume of plasma collected in the transfusion plasma collection container 42 corresponds to a target plasma volume.

In general, during the plasma collection stage, the controller is configured to actuate the pump system and the valve system to convey blood from the whole blood container 50 into the processing chamber 54, which interacts with the centrifuge 22. The controller also actuates the centrifuge 22 to separate the blood in the processing chamber 54 into at least plasma and red blood cells. The pump system is also actuated to convey the separated plasma from the processing chamber 54 through the first outlet and to convey the separated red blood cells from the processing chamber 54 through the second outlet for collection in the red blood cell collection container 46.

As described further below, in the transfusion collection plasma step of the plasma collection stage, the valve system is actuated to convey a first volume of separated plasma to the transfusion plasma collection container 42. In the fractionation plasma collection step of the plasma collection stage, the valve system is actuated to convey a second volume of separated plasma to the fractionation plasma collection container 44.

### "Transfusion Plasma Collection" Step

In the transfusion plasma collection step illustrated in Figs. 6 and 7, the centrifuge 22, the whole blood pump 16, and the plasma pump 18 all continue operating at the same rates at which they were operating at the end of the previous stage (e.g., the establish separation stage of Fig. 5). However, the valve system of the processing device 10 is adjusted to direct the separated plasma and red blood cells to their respective collection containers, rather than recombining them and recirculating them through the centrifuge 22 (as in the establish separation stage).

In particular, in this step, valve 38c is closed which causes the whole blood pump 16 to draw additional blood into line L1 from the whole blood container 50. The whole blood pump 16 draws the blood from the whole blood container 50 into line L2 from line L1, with the blood passing through air trap 62, pressure sensor 40a, and optical sensor 34 before flowing into the processing chamber 54, where it is separated into at least plasma and red blood cells, and optionally a buffy coat or white blood cell layer, by operating the centrifuge 22 at a rate sufficient for separating the whole blood into such components (e.g., between 4500-5500 rpm).

A first volume of separated plasma exits the processing chamber 54 via the first outlet, and associated line L3. Valve 38a and clamp 24b (which may also be referred to herein as a "second valve") are closed, which directs the first volume of plasma from line L3 into line L7, through open clamp 24c (which may also be referred to herein as a "first valve"), and into the transfusion plasma collection container 42. Thus, in this step, the pump system and valve system are actuated to convey the first volume of separated plasma from the processing chamber 54 into the transfusion plasma collection container 42 via a flow path formed by lines L3 and L7 in the illustrated example, i.e., the first flow path.

Further, in this step, the separated red blood cells exit the processing chamber 54 via the second outlet and associated line L4. The additive pump 20 is operated by the controller to draw an additive solution (which may be ADSOL^{®} or some other red blood cell additive) from the additive solution container 48 via line L10. The red blood cells flowing through line L4 are mixed with the additive solution flowing through line L10 at a junction of the two lines L4 and L10 to form a mixture that continues flowing into and through line L5. The mixture is ultimately directed into the red blood cell collection container 46. In the illustrated example of Fig. 6, the mixture is optionally conveyed into line L11, through open valve 38d and an optional leukoreduction filter 64, before flowing through line L12 and open clamp 24a into the red blood cell collection container 46.

Alternatively, as shown in Fig. 7, the valve system may be actuated to cause the mixture to bypass the leukoreduction filter 64 (via lines L8 and L13) and enter the red blood cell collection container 46 without being leukoreduced. For example, the valve system may be controlled to close valve 38d and open valve 38b, such that the mixture is directed from line L5 into line L8, and then into line L13 through the open valve 38b. The mixture may then be directed into line L12, through open clamp 24a and into red blood cell collection container 46.

It is also within the scope of the present disclosure for the mixture to be routed through the leukoreduction filter 64 at the beginning of the transfusion plasma collection step (Fig. 6), with the valve system being reconfigured during the transfusion plasma collection step to cause the mixture to bypass the leukoreduction filter 64 (Fig. 7), such that only a portion of the collected red blood cells is leukoreduced. In one embodiment, pressure sensor 40b monitors the pressure of the leukoreduction filter 64. If the pressure sensor 40b detects that the pressure of the leukoreduction filter 64 has risen above a predetermined pressure threshold (which may be indicative of filter blockage), the controller may reconfigure the valve system to cause the mixture to bypass the leukoreduction filter 64 via lines L8 and L13 and open valve 38b. The system may then alert the operator that the red blood cell product was not leukoreduced.

Thus, in the transfusion plasma collection step, the pump system and valve system may be actuated to convey the separated red blood cells from the processing chamber 54 into the red blood cell collection container 46. In one example, as shown in Fig. 6, the valve system and pump system may be actuated to convey the red blood cells (including red blood cells mixed with the additive solution) along a flow path formed by lines L4, L5, L11 and L12, such that the red blood cells are flowed through the leukoreduction filter 64 before reaching the red blood cell collection container 46. Alternatively, as shown in Fig. 7, the valve system and the pump system may be actuated to convey the red blood cells (including red blood cells mixed with the additive solution) along a flow path formed by lines L4, L5, L8, L13 and L12 to bypass the leukoreduction filter 64 before reaching the red blood cell collection container 46.

The transfusion plasma collection step may be performed until the volume of plasma collected in the transfusion plasma collection container 42 (i.e., the first volume of separated plasma) corresponds to a target plasma volume. In various embodiments, the controller may determine the volume of plasma in the transfusion plasma collection container 42 by monitoring a weight of the transfusion plasma collection container 42 and/or a volume of plasma moved by the plasma pump 18.

For example, the processing device 10 may include a transfusion plasma sensor (not shown) configured to detect a transfusion plasma status and transmit transfusion plasma status information indicative of the detected transfusion plasma status to the controller. The controller may determine the volume of plasma collected in the transfusion plasma collection container 42 (i.e., the first volume) based on the transfusion plasma status information. In one embodiment, the transfusion plasma sensor may be a weight sensor and the transfusion plasma status may be a weight of the transfusion plasma collection container 42. The weight sensor may transmit weight information indicative of the detected weight of the transfusion plasma collection container 42 to the controller and the controller may determine the volume of plasma collected in the transfusion plasma collection container 42 based on the weight information.

Alternatively, or in addition, the transfusion plasma sensor may be a flow sensor and the transfusion plasma status may be a volume flow rate of plasma moved by the plasma pump 18. The flow sensor may transmit flow information indicative of the detected volume flow rate of plasma moved by plasma pump 18 to the controller and the controller may determine the volume of plasma collected in the transfusion plasma collection container 42 based on the flow information.

The controller may then monitor the volume of plasma in the transfusion plasma collection container 42 and determine when the volume of plasma in the transfusion plasma collection container 42 corresponds to a target plasma volume. In one example, the target plasma volume may be 180 mL, which corresponds to a typical minimum volume of plasma for a transfusion plasma product unit. Thus, plasma may be collected in the transfusion plasma collection container 42 until the controller determines the volume of collected plasma corresponds to (e.g., is equal to) the target plasma volume. Accordingly, a transfusion plasma product may be produced in the manner above having a volume which meets minimum volume requirements for transfusion plasma products without exceeding (within tolerances), the minimum volume requirements for the plasma product.

In response to determining the volume of plasma in the transfusion plasma collection container 42 corresponds to the target plasma volume, the controller may be configured to control the valve system to end the transfusion plasma collection step and to initiate the fractionation plasma collection step.

### "Fractionation Plasma Collection" Step

Referring now to Figs. 8 and 9, in the fractionation plasma collection step of the plasma collection stage, the centrifuge 22, the whole blood pump 16, the plasma pump 18 and the additive pump 20 all continue operating at the same rates at which they were operating at the end of the transfusion plasma collection step (Figs. 6 and 7). However, the valve system of the processing device 10 is adjusted to direct the separated plasma to the fractionation plasma collection container 44, rather than the transfusion plasma collection container 42.

In particular, in this step, valve 38c remains closed such that whole blood pump 16 continues to draw additional blood into line L1 from the whole blood container 50. The whole blood pump 16 draws the whole blood into line L2 from line L1, with the blood passing through air trap 62, pressure sensor 40a and optical sensor 34 before flowing into the processing chamber 54. The centrifuge 22 continues to operate such that the blood in the processing chamber 54 is separated into plasma and red blood cells.

A second volume of separated plasma exits the processing chamber 54 via the first outlet and associated line L3. Valve 38a and clamp 24c (i.e., first valve 24c) are closed, which directs the plasma from line L3 into line L16, through open clamp 24b (i.e. second valve 24b), and into the fractionation plasma collection container 44. Thus, in this step, the pump system and valve system are actuated to convey the second volume of separated plasma from the processing chamber 54 into the fractionation plasma collection container 44 via a flow path including lines L3 and L16, which may be referred to herein as a second flow path.

In this step, the separated red blood cells exit the processing chamber 54 in the same manner as in the transfusion plasma collection step. Thus, the separated red blood cells continue to exit the processing chamber 54 via the second outlet and associated line L4 and are combined with the additive solution drawn from the additive solution container 48 by operation of additive pump 20 to form a mixture in line L5. The mixture is ultimately directed into the red blood cell collection container 46. In the illustrated example of Fig. 8, the mixture is optionally conveyed into line L11, through open valve 38d and optional leukoreduction filter 64 before flowing through line L12 and open clamp 24a into the red blood cell collection container 46.

Alternatively, as shown in Fig. 9, the valve system may be actuated to cause the mixture to bypass the leukoreduction filter 64 (via lines L8 andL13) and enter the red blood cell collection container 46 without being leukoreduced. For example, the valve system may be controlled to close valve 38d and open valve 38b, such that the mixture is directed from line L5 into line L8, and then into line L13 through the open valve 38b. The mixture may then be directed into line L12, through open clamp 24a and into red blood cell collection container 46.

It is also within the scope of the present disclosure for the mixture to be routed through the leukoreduction filter 64 at the beginning of the fractionation plasma collection step (Fig. 8), with the valve system being reconfigured during the fractionation plasma collection step to cause the mixture to bypass the leukoreduction filter 64 (Fig. 9), such that only a portion of the collected red blood cells is leukoreduced. In one embodiment, pressure sensor 40b monitors the pressure of the leukoreduction filter 64. If the pressure sensor 40b detects that the pressure of the leukoreduction filter 64 has risen above a predetermined pressure threshold (which may be indicative of filter blockage), the controller may reconfigure the valve system to cause the mixture to bypass the leukoreduction filter 64 via lines L8 and L13 and open valve 38b. The system may then alert the operator that the red blood cell product was not leukoreduced.

Thus, in the fractionation plasma collection step, the pump system and the valve system may be actuated to convey the separated red blood cells from the processing chamber 54 into the red blood cell collection container 46. In one embodiment, as shown in Fig. 8, the valve system and the pump system may be actuated to convey the red blood cells (including red blood cells mixed with the additive solution) along a flow path including lines L4, L5, L11 and L12, such that the red bloods are flowed through the leukoreduction filter 64 before reaching the red blood cell collection container 46. Alternatively, as shown in Fig. 9, the valve system and the pump system may be actuated to convey the red blood cells (including red blood cells mixed with the additive solution) along a flow path formed by lines L4, L5, L8, L13 and L12 to bypass the leukoreduction filter 64 before reaching the red blood cell collection container 46.

The fractionation plasma collection step may be performed until the volume of blood in the whole blood container 50 is drawn down a target blood volume. For example, the fractionation plasma collection step may be performed until the whole blood container 50 is substantially emptied, i.e., until the volume of blood in the whole blood container 50 is approximately zero (within tolerances). However, it will be appreciated that the target blood volume may be a non-zero volume in other examples.

For example, the processing device 10 may include a blood supply sensor configured to detect a blood supply status and transmit blood supply status information indicative of the detected blood supply status to the controller. The controller may determine the volume of blood remaining in the blood supply container 50 based on the blood supply status information. In one embodiment, the blood supply sensor may be a weight sensor and the blood supply status may be a weight of the blood supply container 50. The weight sensor may transmit weight information indicative of the detected weight of the blood supply container 50 to the controller and the controller may determine the volume of blood remaining in the blood supply container 50 based on the weight information.

Alternatively, or in addition, the blood supply sensor may be a pressure sensor, such as pressure sensor 40c, configured to detect a hydrostatic pressure in a line (e.g., line L1) downstream from the blood supply container 50. The pressure sensor may transmit pressure information indicative of the detected hydrostatic pressure to the controller and the controller may determine the volume of blood remaining in the blood source container 50 based on the pressure information.

The controller may then monitor the volume of blood in the whole blood container 50 and determine when the volume of blood in the container 50 corresponds to the target blood volume. In one example, the target blood volume may be approximately 0 mL, indicating the blood source container 50 is substantially empty. Thus, after a first volume of separated plasma is collected in the transfusion plasma collection container 42 to provide the transfusion plasma product, a remaining volume of whole blood in the whole blood container 50 may continue to be drawn into the processing chamber 54 until the controller determines the remaining volume of blood in the container 50 corresponds to (e.g., is equal to) the target blood volume (which may be indicative of an empty blood supply container 50). The remaining volume of blood may be separated in the processing chamber 54 into at least the second volume of separated plasma and red blood cells. The second volume of separated plasma may then be collected in the fractionation plasma collection container 44 to produce a fractionation plasma product. The fractionation plasma product may be valued per unit volume and can be provided separately from the transfusion plasma product.

Accordingly, the plasma collection stage, a single unit of whole blood may be separated to produce a transfusion plasma product having a first predetermined volume (such as a 180 mL) corresponding to a minimum volume requirement for a transfusion plasma product, and a fractionation plasma product which includes plasma separated from the remaining portion whole blood in the whole blood container 50. The transfusion plasma product may be sold at a per unit price without exceeding minimum volume requirements for the transfusion plasma product, while the fraction plasma product may be sold at a per unit volume price.

In this manner, a volume of plasma exceeding a minimum volume requirement (e.g., a volume in excess of 180 mL for a transfusion plasma product) may be collected in a separate container and provided as a separate plasma product (i.e., a fractionation plasma product) to be sold at a per unit volume value. In this manner, excess volumes of plasma which may have been included in previous transfusion plasma products and which did not increase a value of the previous transfusion plasma product, may now be separately collected according to the present plasma collection techniques, and ultimately sold as a fractionation plasma product on a per unit volume basis. Thus, greater value may be realized from plasma products derived from a single unit of whole blood using the present plasma collection stage than previous plasma products derived from a single unit of whole blood using previous plasma collection techniques.

### "Red Blood Cell Recovery" Stage

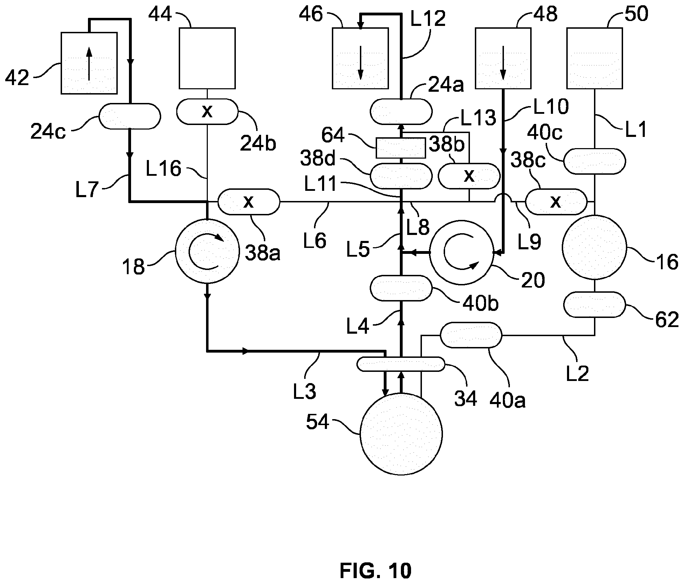

The plasma collection stage continues until the remaining volume of blood in the whole blood container 50 corresponds to the target blood volume, or until a target volume of blood has been drawn into the fluid flow circuit 12 from the whole blood container 50. The controller may then transition the procedure to an optional "red blood cell recovery" stage illustrated in Fig. 10. During the red blood cell recovery stage, air from the transfusion plasma collection container 42 (which was conveyed there when priming the fluid flow circuit 12) is used to recover separated red blood cells remaining in the processing chamber 54 (optionally without removing the buffy coat or white blood cell layer from the processing chamber 54).

In the illustrated embodiment, the whole blood pump 16 is deactivated and the additive pump 20 remains activated from the plasma collection stage. The plasma pump 18 is operated in a reverse direction with respect to its direction of operation up to this stage of the procedure. This draws the air from the transfusion plasma collection container 42 into line L7. Clamp 24b and valve 38a are closed, while clamp 24c is open, which directs the air through line L7, into and through line L3, and into the processing chamber 54 via the plasma outlet port. On account of the air flowing through the plasma outlet port, it will enter the processing chamber 54 at the low-g side. As additional air is introduced into the processing chamber 54, it will move from the low-g wall towards the high-g wall, thus displacing the separated red blood cells in the processing chamber 54 through the red blood cell outlet port at the high-g side and into line L4. During this stage, the centrifuge 22 may be operated at a slower rate (e.g., in the range of approximately 1,000-2,000 rpm) to decrease the risk of an air blockage.

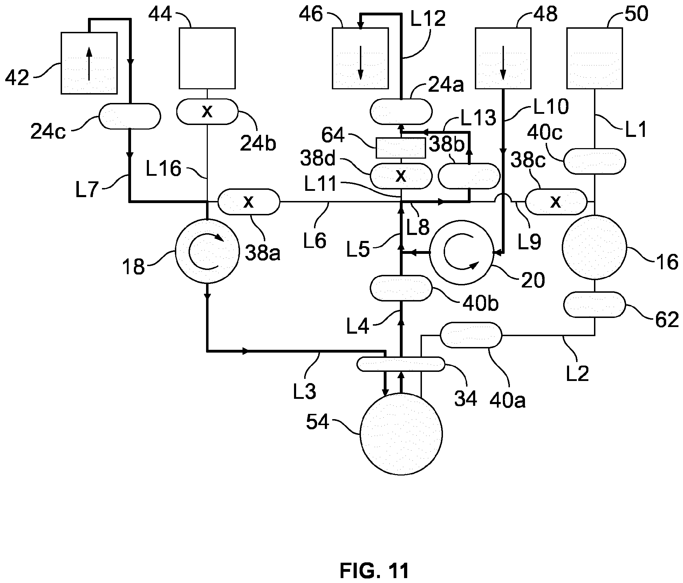

The additive pump 20 operates to draw additive solution from the additive solution container 48 and through line L10, to be mixed with the red blood cells flowing through line L4 at the junction of the two lines L4 and L10. The mixture continues flowing into and through line L5. If, at the end of the plasma collection stage, the valve system was arranged so as to direct flow through the leukoreduction filter 64, then valves 38a, 38b, and 38c may remain closed, with valve 38d being open to direct the mixture into line L11 for leukoreduction, as shown in Fig. 10. On the other hand, if the valve system was arranged so as to bypass the leukoreduction filter 64 at the end of the plasma collection stage, then valves 38a, 38c, and 38d may remain closed, with valve 38b being open to direct the mixture through lines L8 and L13 to bypass the leukoreduction filter 64 (Fig. 11). As described above with regard to the plasma collection stage, it is possible for the controller to change the configurations of the valve system during the red blood cell recovery stage to stop leukoreduction of the mixture (e.g., if the pressure of the leukoreduction filter 64 becomes too great).

Regardless of whether the mixture is filtered, it flows into line L12, through open clamp 24a, and into the red blood cell collection container 46. The red blood cell recovery stage continues until the red blood cells have been removed from the processing chamber 54. This may be determined in any of a number of ways without departing from the scope of the present disclosure. In one embodiment, the red blood cell recovery stage continues until a predetermined volume of fluid (corresponding to the volume of red blood cells remaining in the processing chamber 54) has been conveyed out of the processing chamber 54. This volume may be calculated for example, by determining the volume of red blood cells present in the whole blood that has been processed (which may be determined based on the hematocrit of the blood) and then subtracting the volume of red blood cells that have already been conveyed into the red blood cell collection container 46 (which may be determined based on the weights of the red blood cell container and the additive solution container 48 at the end of the plasma collection stage). In another embodiment, the red blood cell recovery stage may continue until the optical sensor 34 detects a non-red blood cell fluid (e.g., the buffy coat or white blood cell layer) flowing through line L4.

Thus, in the red blood cell recovery stage, the controller may be configured to actuate the pump system and the valve system to convey air from the transfusion plasma collection container 42 into the processing chamber 54 via lines L7 and L3. The centrifuge may be actuated such that the air urges the separated red blood cells remaining in the processing chamber 54 toward the second outlet and associated line L4. The pump system and valve system may be actuated to convey the separated red blood cells from the processing chamber 54 into the red blood cell collection container 46 via flow path which may be referred to herein as a fourth flow path, and which may include lines L4, L5, L11 and L12 to pass through the leukoreduction filter 64, or lines L4, L5, L8, L13 and L12 to bypass the leukoreduction filter 64.

### "Additive Solution Flush" Stage

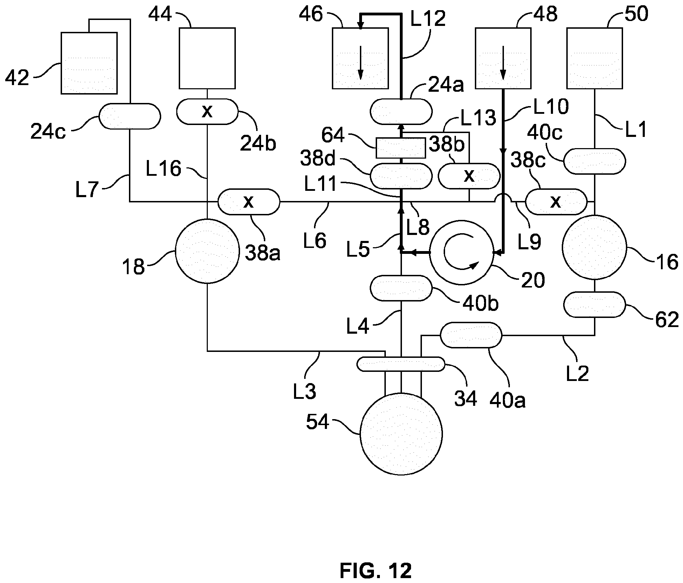

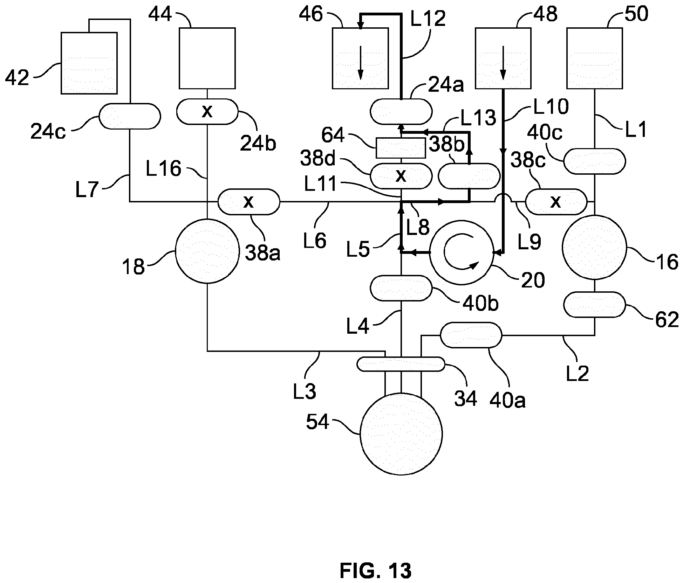

Once the red blood cell recovery stage is complete, the procedure may transition to an optional "additive solution flush" stage as illustrated in Figs. 12 and 13. During the additive solution flush stage, additive solution from the additive solution container 48 is conveyed into the red blood cell collection container 46 until a target amount of additive solution is in the red blood cell collection container 46.

To transition from the red blood cell recovery stage to the additive solution flush stage, clamps 24b and 24c are closed, clamp 24a is opened, the whole blood pump 16 and plasma pump 18 are deactivated, and the additive pump 20 is activated. Additionally, one of valves 38b and 38d is opened, which determines whether the additive solution will be conveyed through the leukoreduction filter 64 via line L11, as shown in Fig. 12, or bypass the leukoreduction filter 64 (via lines L8 and L13) as shown in Fig. 13, and which may depend on which of the two valves 38b and 38d was open at the end of the red blood cell recovery stage. Typically, if valve 38d was open at the end of the red blood cell recovery stage (as in Fig. 10), then it will be opened at the beginning of the additive solution flush stage (as in Fig. 12) to allow for red blood cells remaining in the leukoreduction filter 64 to be recovered by additive solution being conveyed through the leukoreduction filter 64. Similarly, if valve 38b was open at the end of the red blood cell recovery stage, as in Fig. 11, then it will be opened at the beginning of the additive solution flush stage to direct additive solution around the leukoreduction filter 64, as in Fig. 13. However, it is also within the scope of the present disclosure for valve 38b to be closed and valve 38d to be open at the end of the red blood cell recovery stage (as in Fig. 10), with valve 38b being open and valve 38d being closed at the beginning of the additive solution flush stage (as in Fig. 13), if the controller determines that it is advisable to begin bypassing the leukoreduction filter 64. Additionally, it is within the scope of the present disclosure for the valve system to be arranged as in Fig. 12 at the beginning of the additive solution flush stage (to direct additive solution through the leukoreduction filter 64) and to transition into a bypass configuration before the end of the additive solution flush stage, as in Fig. 13.

Thus, in the additive solution flush stage, the controller may be configured to actuate the pump system and the valve system to convey additive solution from the additive solution container 48 into the red blood cell collection container. In one example, as shown in Fig. 12, the additive solution may be conveyed along a flow path including lines L10, L5, L11 and L12 to pass through the leukoreduction filter 64 before reaching the red blood cell collection container 46. In another example, as shown in Fig 13, the additive solution may be conveyed along a flow path including lines L10, L5, L8, L13 and L12 to bypass the leukoreduction filter 64 before reaching the red blood cell collection container 46.

### "Air Evacuation" Stage

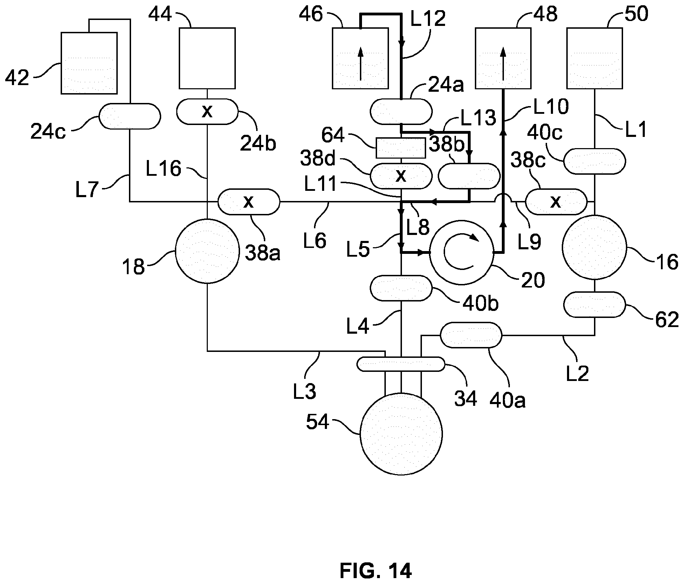

The additive solution flush stage will continue until a target amount of additive solution has been added to the red blood cell collection container 46. When the additive solution flush stage is complete, the system may transition to an optional "air evacuation" stage (sometimes referred to as a red blood cell "burp" stage), as shown in Fig. 14. During the air evacuation stage, the red blood cell collection container 46 is "burped" to remove all residual air for storage (just as air may be removed from the transfusion plasma collection container 42 during the red blood cell recovery stage. This is done by reversing the direction of operation of the additive pump 20, closing valve 38d (if not already closed at the end of the additive solution flush stage), and opening valve 38b (if not already open at the end of the additive solution flush stage). The additive pump 20 draws air out of the red blood cell collection container 46 and into the additive solution container 48. While Fig. 14 shows the air being evacuated from the red blood cell collection container 46 to the additive solution container 48, it is within the scope of the present disclosure for all or a portion of the air to be directed to a different location of the fluid flow circuit 12.

The air evacuation stage will continue until all of the air is removed from the red blood cell collection container 46. Upon completion of the air evacuation stage, any of a number of post-processing stages may be executed. For example, a "sealing" stage in which all of the clamps and valves are closed and all of the pumps are deactivated may be executed. The line L12 connected to the red blood cell collection container 46, the line L7 connected to the transfusion plasma collection container 42, and the line L16 connected to the fractionation plasma collection container 44 are sealed and optionally severed for storage of the transfusion plasma, red blood cell, and fractionation plasma products. Optionally, air may be removed from the fractionation plasma collection container 44 (e.g., using a variation of the air evacuation stage of Fig. 14) before sealing line L16.

It should be understood that the processing device 10 shown in Fig. 1, the fluid flow circuit 12 shown in Figs. 2 and 3, and the procedure shown in Figs. 4-14 are merely exemplary of a system and procedure that may employ the techniques and principles described herein. Indeed, differently configured blood separation systems and separation procedures including different steps may employ the techniques and principles described herein without departing from the scope of the present disclosure.

It will be appreciated that the present disclosure is not limited to the collection of red blood cells, transfusion plasma and fractionation plasma. For example, in other embodiments, the system of the present disclosure may be configured to additionally collect one buffy coat, and/or various combinations of separated blood constituents along with the transfusion plasma and fractionation plasma. In addition, it will be appreciated that although continuous flow centrifugation is referenced in the examples above, that other separation devices capable of producing platelet poor plasma and packed red blood cells, such as a spinning membrane, may be implemented in present disclosure as well.

### Centrifuge Orientation

According to an aspect of the present disclosure, the separation device 10 and the fluid flow circuit 12 may be used in combination to execute a procedure having a modified "priming" stage (which may be understood as a variation of the above-described "priming" stage) and/or an improved "air harvest" stage (which may be understood as a variation of the above-described "red blood cell recovery" stages). Any intermediate stages may be executed between the "priming" stage (which takes place before fluid separation) and the "air harvest" stage (which takes place after fluid separation) without departing from the scope of the present disclosure. Thus, it should be understood that a separation procedure according to the present disclosure may include a "priming" stage of the type described herein and not an "air harvest" stage of the type described herein, an "air harvest" stage of the type described herein and not a "priming" stage of the type described herein, or both a "priming" stage and an "air harvest" stage of the type described herein, along with any other fluid separation stages (e.g., the above-described "plasma collection" stage).

Notably, in contrast to the version of the "priming" stage described above (in which the centrifuge 22 is spun at a low rotation rate), in this modified version, the centrifuge 22 is stationary or inoperative during the "priming" stage, with the separation chamber 54 being oriented so as to position outlet ports of the chamber 54 at a "high point" of the chamber 54 (as will be described in greater detail). Air within the separation chamber 54 will naturally move toward the "high point" of the chamber 54, which aids in removing the air from the chamber 54 during the "priming" stage, which otherwise may proceed as described above for the "priming" stage of Fig. 4.

Later, after fluid separation has been completed (e.g., after the above-described "plasma collection" stage), the "air harvest" stage may begin. During this stage, air is used to recover separated fluid components remaining within the separation chamber 54 after fluid separation has been completed. In one embodiment of such a stage, operation of the plasma pump 18 and the whole blood pump 16 will draw air from container 42 into the separation chamber 54. The air will enter the separation chamber 54 via an outlet port of the chamber 54 and displace the fluid and/or fluid components remaining in the chamber 54 through the inlet port and into line L2. The fluid and/or fluid components are directed from line L2 into line L1 and then into container 50 or, alternatively, from line L2 into line L9 and into container 46 (via lines L8, L11, and L12, if the fluid and/or fluid components are to be leukoreduced, or via lines L13 and L12, if the fluid and/or fluid components are not to be leukoreduced). In another embodiment of an "air harvest" stage (which is more similar to the above-described "red blood cell recovery" stages), rather than the fluid and/or fluid components exiting the separation chamber 54 via line L2, the fluid and/or fluid components may instead exit the separation chamber 54 via line L4 and the associated outlet port.

Notably, in contrast to the "red blood cell recovery" stages described above (in which the centrifuge 22 is spun at a low rotation rate), the centrifuge 22 is stationary or inoperative during the "air harvest" stage, with the separation chamber 54 being oriented so as to position an inlet port of the chamber 54 (or whichever port is used to direct the fluid and/or fluid components from the chamber 54) at a "low point" of the chamber 54 (as will be described in greater detail). Air within the separation chamber 54 will naturally move toward the "high point" of the chamber 54, with the fluid and/or fluid components moving toward the "low point," which aids in recovering the fluid and/or fluid components from the chamber 54 during the "air harvest" stage.

The "air harvest" stage continues until the fluid and/or fluid components have been removed from the separation chamber 54. This may be determined in any of a number of ways without departing from the scope of the present disclosure. In one embodiment, the "air harvest" stage continues until a predetermined volume of fluid (corresponding to the volume of the fluid and/or fluid components in the separation chamber 54) has been conveyed out of the chamber 54. In another embodiment, the "air harvest" may continue until the optical sensor 34 detects air flowing through line L2 (or whichever conduit is configured to receive flow of the fluid and/or fluid components exiting the separation chamber 54). Once the "air harvest" stage is complete, the procedure may end or transition to any other stage of the procedure (e.g., a stage in which an additive fluid is added to one of the collected fluid components, such as the "additive solution flush" stage described above). An "air harvest" stage according to this aspect of the present disclosure may be advantageous to the extent that it allows for more complete removal of residual fluid and/or fluid components from the separation chamber 54 than conventional approaches, which may leave small amounts of the fluid and/or fluid components in the chamber 54 that are ultimately discarded with the flow circuit as waste.

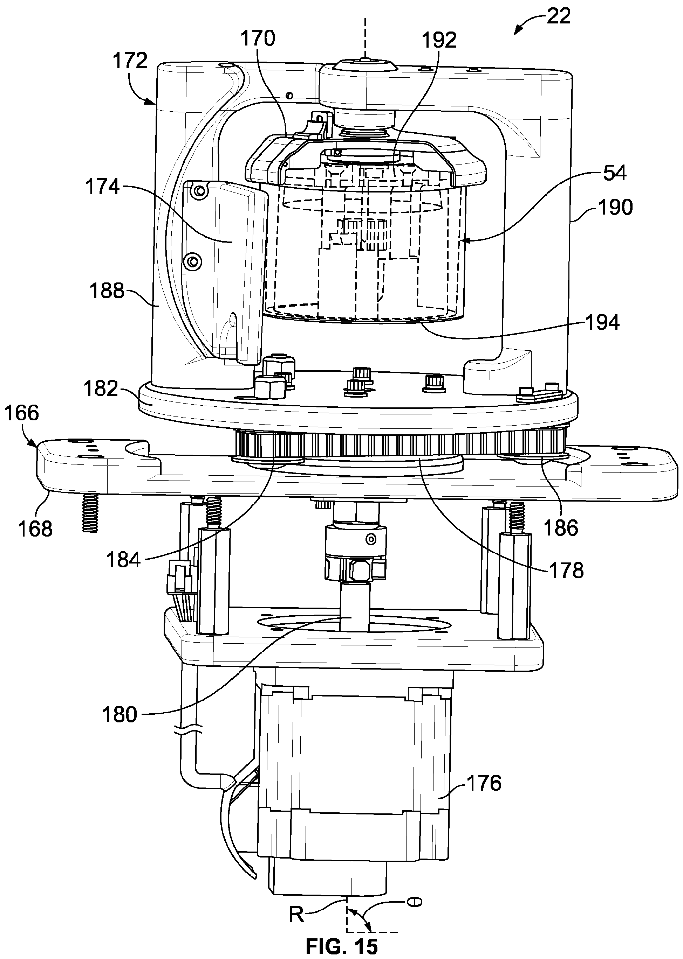

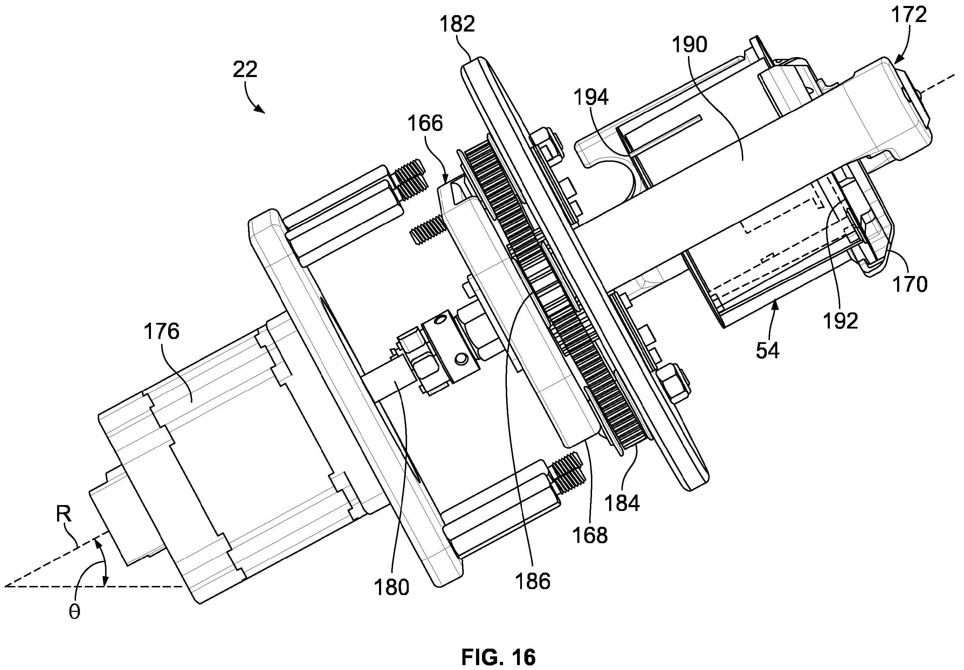

Figs. 15 and 16 illustrate components of an exemplary embodiment of a centrifuge 22 that may be used to implement the improved "priming" and "air harvest" stages, in which a separation chamber is moved into target orientations. A centrifuge 22 having a direct drive assembly of the type shown in Figs. 15 and 16 is particularly well-suited to determination of the orientation of a separation chamber and movement of the chamber into a target orientation. It should be understood that the direct drive assembly shown in Figs. 15 and 16 is merely exemplary and that differently configured direct drive assemblies may be employed without departing from the scope of the present disclosure.

As used herein, the phrase "direct drive" refers to an arrangement in which rotation of a motor of a centrifuge is imparted to a separation chamber mounted to the centrifuge via a mechanical coupling rather than an arrangement in which rotation of a centrifuge motor is imparted to a separation chamber by an umbilicus of a fluid flow circuit, as in the system described in PCT Patent Application Publication No. WO 2021/194824 A1. In "umbilicus-driven" centrifuges, a motor rotates a support at a "one-omega" speed, with the support engaging a midsection of the umbilicus. Rotation of the support causes the umbilicus to orbit around an associated separation chamber at the "one-omega" speed, with the movement of the umbilicus causing the separation chamber to rotate about a rotational axis. The midsection of the umbilicus is allowed to rotate with respect to the support, while one end of the umbilicus is held in place by a component of the centrifuge and prevented from rotating, which causes the umbilicus to become twisted. Periodically, the umbilicus will react to this by resiliently returning to its untwisted condition, with the separation chamber being rotated at an increased rate at this time. This behavior of the umbilicus causes the separation chamber to be rotated at an average speed of "two-omega," which is twice the rate at which the centrifuge motor operates.

While an "umbilicus-driven" centrifuge is capable of rotating a separation chamber at an appropriate speed for fluid separation, it may be impracticable to place the separation chamber in a desired or target orientation within the centrifuge. More particularly, in an "umbilicus-driven" centrifuge, a centrifuge motor may be controlled so as to rotate the midsection of the umbilicus to a target position. While the position of the midsection of the umbilicus is indicative or suggestive of the orientation of the separation chamber, due to the nature of the umbilicus as a flexible, deformable component, the exact orientation of the separation chamber cannot be controlled by operation of the centrifuge motor. In contrast, in a direct drive system, the exact orientation of the separation chamber may be determined by a controller and adjusted by operation of the centrifuge motor. Accordingly, it may be advantageous for a fluid separation device according to the present disclosure to employ a centrifuge having a direct drive assembly.

Turning back now to the exemplary centrifuge configuration shown in Figs. 15 and 16, the centrifuge 22 includes a stationary frame 166 having a lower plate 168 spaced from an upper support (not illustrated) that is used to secure a first end of an umbilicus so as to prevent rotation of that end of the umbilicus. The separation chamber 54 (which is secured to a second end of the umbilicus, as shown in Fig. 2) is mounted or secured to a receptacle 170 of the centrifuge 22. A yoke 172 of the centrifuge 22 includes a central support 174 configured to engage a midsection of the umbilicus, allowing the midsection of the umbilicus to rotate with respect to the central support 174.

A motor 176 is secured to the lower plate 168 of the frame 166. A sun gear 178 is fixedly secured with respect to the lower plate 168. A drive shaft 180 extends through the lower plate 168 and the sun gear 178, with the drive shaft 180 defining a rotational axis "R" of the yoke 172. The motor 176 is controlled by a controller of the separation device 10 to rotate the drive shaft 180 at a desired "one-omega" speed. The selected "one-omega" speed is based on the rate at which the separation chamber 54 is to be rotated with respect to the stationary frame 166. More particularly, the "one-omega" speed is half the "two-omega" speed at which the separation chamber 54 rotates with respect to the frame 166 so, if it desired for the separation chamber 54 to rotate at 4,500 RPM with respect to the frame 166, the controller will command the motor 176 to operate so as to rotate the drive shaft 180 at a "one-omega" speed of 2,250 RPM.

The drive shaft 180 is secured with respect to a bottom platform 182 of the yoke 172, such that rotation of the drive shaft 180 will cause the bottom platform 182 (and the remainder of the yoke 172) to rotate at the same speed (i.e., the "one-omega" speed). As can be seen in Figs. 15 and 16, the lower plate 168 of the frame 166 is spaced from the bottom platform 182 of the yoke 172, with at least a portion of the sun gear 178 and an associated timing belt 184 positioned therebetween. In addition to engaging the stationary sun gear 178, the timing belt 184 also engages a planet gear 186 that is oriented in the same plane as the sun gear 178.

The illustrated yoke 172 includes two arms 188 and 190, with the central support 174 being associated with the first arm 188. On account of the central support 174 of the yoke 172 engaging the midsection of the umbilicus, the midsection of the umbilicus will orbit about the rotational axis R of the yoke 172 at the same "one-omega" rotational speed, while the midsection of the umbilicus is allowed to rotate about its own central axis with respect to the central support 174. As for the second arm 190 of the yoke 172, a yoke shaft extending from the planet gear 186 is rotatably received therewithin (and oriented substantially parallel to the drive shaft 180).

As noted above, the timing belt 184 engages the planet gear 186, such that rotation of the planet gear 186 with the bottom platform 182 of the yoke 172 also causes the timing belt 184 to rotate about the sun gear 178. As the sun gear 178 is stationary (while the planet gear 186 is free to rotate about its own central axis with respect to the bottom platform 182 of the yoke 172), rotation of the timing belt 184 about the sun gear 178 will cause the planet gear 186 to rotate with respect to the bottom platform 182.

The yoke shaft extends from a first end associated with the planet gear 186 to a second end associated with a sheave that is also rotatably positioned within the yoke 172. The sheave is engaged by a second timing belt, which also engages a second sheave that is positioned in a common plane with the first sheave and is rotatably positioned within the yoke 172. The receptacle 170 (which is at least positioned outside of the yoke 172 so as to be able to be connected to the separation chamber 54) is configured to rotate with the second sheave, with both being substantially coaxial with the drive shaft 180, the sun gear 178, and the upper support, along the rotational axis R of the yoke 172.

Rotation of the planet gear 186 causes the yoke shaft and the first sheave to rotate at the same rate, as a unitary structure. The second timing belt transfers the rotation of the first sheave to the second sheave, with the receptacle 170 and separation chamber 54 rotating at the same rate as the second sheave. By employing an appropriate set of gears and/or belts and/or sheaves, driving the motor 176 at the "one-omega" speed causes the second sheave and, thus, the receptacle 170 and separation chamber 54 to rotate at the proper "two-omega" speed that is required for fluid separation. Additional details of a suitably configured direct drive assembly can be found in U.S. Patent Application Serial No. 18/539,366, which is hereby incorporated herein by reference. However, it is again noted that differently configured direct drive assemblies may be employed without departing from the scope of the present disclosure.

Notably, Fig. 16 shows the centrifuge 22 (and, thus, the rotational axis R) in an angled (i.e., non-horizontal, non-vertical) orientation, whereas Fig. 15 shows the centrifuge 22 (and rotational axis R) in a vertical orientation. Fig. 16 shows the centrifuge 22 and rotational axis R oriented at an angle θ from horizontal, which angle θ is also shown in Fig. 1. In one embodiment, the angle θ is in the range of approximately 30° to approximately 60°, but it is within the scope of the present disclosure for the centrifuge 22 and rotational axis R to be oriented at some other angle θ.

As noted above and as will be described in greater detail, improved "priming" and "air harvest" stages require movement of a separation chamber 54 into target orientations in which the separation chamber has a "high point" and a "low point." When the centrifuge 22 is arranged in a vertical orientation, the "high point" of the separation chamber 54 (i.e., the portion of the separation chamber 54 positioned at a maximum vertical elevation) will be the entire upper end or lid 192 of the separation chamber 54, with the entire bottom end or base 194 of the separation chamber 54 being the "low point" of the chamber 54 (i.e., the portion of the separation chamber 54 positioned at a minimum vertical elevation). Thus, when the centrifuge 22 is arranged in a vertical orientation (as in Fig. 15), the same portion of the separation chamber 54 (the upper end or lid 192) will always remain at the "high point" and the same portion of the separation chamber 54 (the bottom end or base 194) will always remain at the "low point," regardless of the orientation or angular position of the separation chamber 54.

In contrast, when the centrifuge 22 and rotational axis R are arranged in a non-horizontal, non-vertical, angled orientation (as in Fig. 16), the orientation or angular position of the separation chamber 54 will determine the portion of the separation chamber 54 located at the "high point" (identified in Fig. 16 at "H") and the portion of the separation chamber 54 located at the "low point" (identified in Fig. 16 at "L"). Thus, when it is desirable for a particular portion of the separation chamber 54 to be positioned at the "high point" H (as is the case during a "priming" stage) or for a particular portion of the separation chamber 54 to be positioned at the "low point" L (as is the case during an "air harvest" stage), the controller may command the centrifuge motor 176 to rotate the separation chamber 54 into the proper, target orientation.

The controller may determine the current orientation of the separation chamber 54 and subsequently control the centrifuge 22 to rotate the separation chamber 54 into a target orientation according to any suitable approach. In one exemplary embodiment, the receptacle 170 of the centrifuge 22 is configured to be secured to the separation chamber 54 via a keyed or asymmetrical connection, which allows for the separation chamber 54 to be mounted to the centrifuge 22 in only one orientation. As the initial orientation of the separation chamber 54 within the centrifuge 22 is known, the controller may control operation of the centrifuge motor 176 to rotate the separation chamber 54 from the initial or current orientation into a target orientation. For example, if the keyed connection between the receptacle 170 and the separation chamber 54 places the separation chamber 54 into an initial orientation that is 30° from a target orientation, the controller may command the motor 176 to operate so as to rotate the separation chamber 54 the required 30° from the initial orientation into the target orientation.

In another embodiment, the optical system of the fluid separation device 10 (which is configured to monitor fluid flow and separation within the separation chamber 54) may be used to determine the current orientation of the separation chamber 54. For example, the separation chamber 54 may be provided with one or more markers or the like that is indicative of the orientation of the separation chamber 54. When the separation chamber 54 has been installed within the centrifuge 22, the optical system may be operated to analyze the separation chamber 54, sending signals to the controller that are indicative of what is present in the line of sight of the optical system. When the controller receives one or more signals indicating that a marker that is positioned within the line of sight of the optical system, it may use such signal(s) to determine the current orientation of the separation chamber 54. Once the controller has determined the current orientation of the separation chamber 54, it may control operation of the centrifuge motor 176 to rotate the separation chamber 54 from the current orientation into a target orientation, as described above with regard to a keyed connection between a separation chamber 54 and the receptacle 170 of the centrifuge 22. Other approaches to determining and controlling the orientation of the separation chamber 54 (e.g., via use of a tachometer feedback system and/or one or more proximity sensors) may also be employed without departing from the scope of the present disclosure.

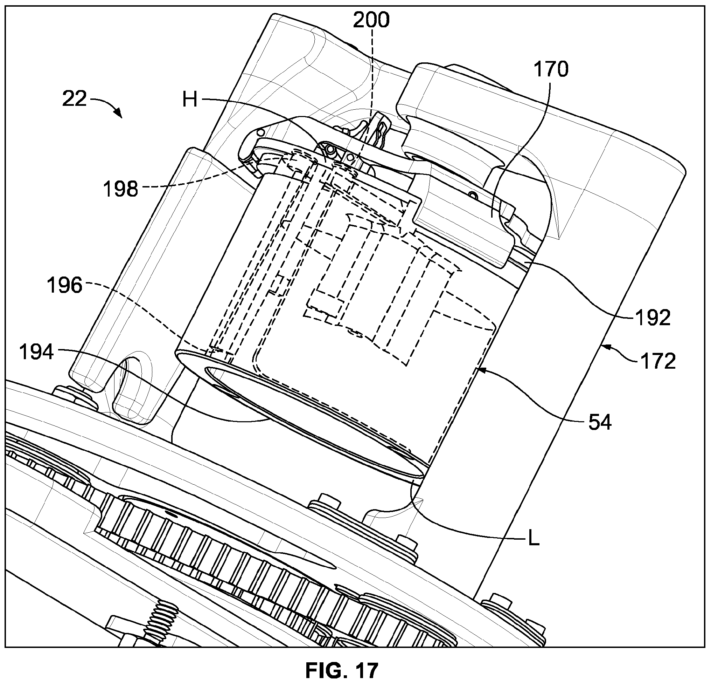

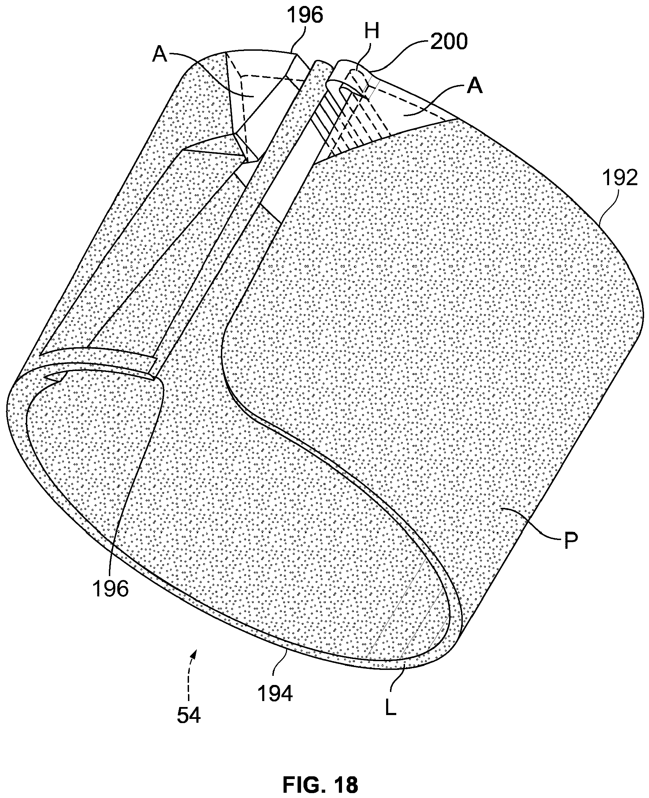

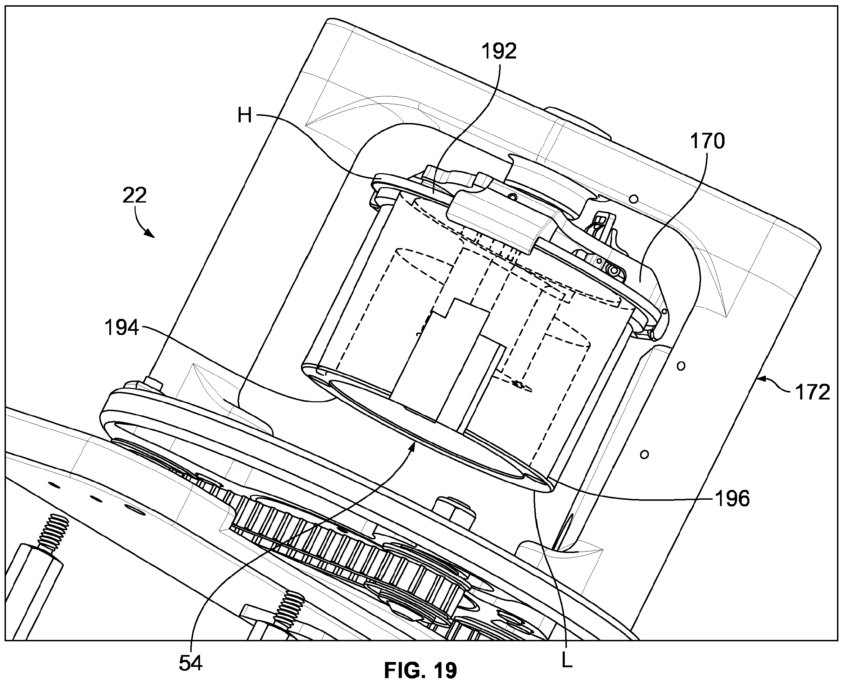

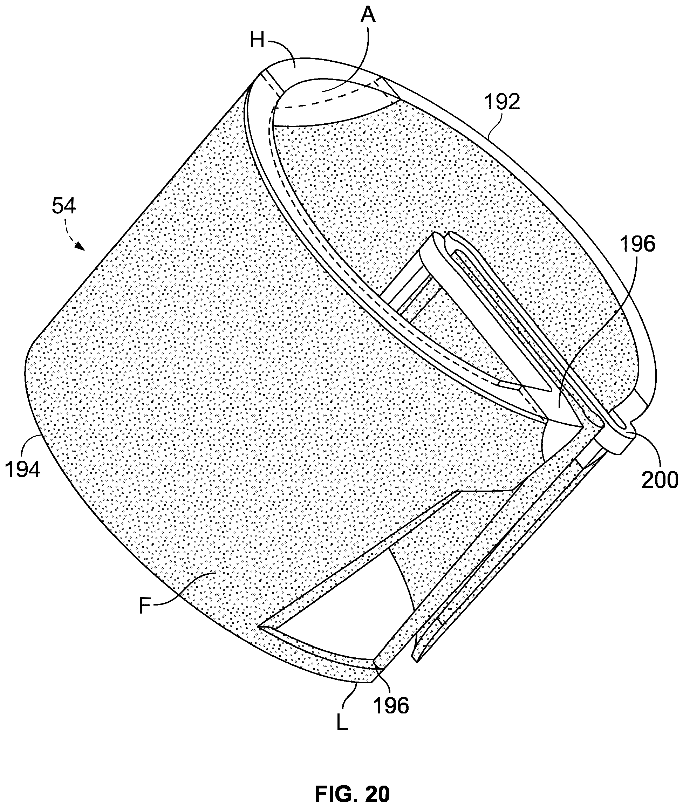

Figs. 17 and 19 respectively illustrate the separation chamber 54 positioned in target orientations for a "priming" stage and an "air harvest" stage, with Fig. 18 illustrating fluid flow within the separation chamber 54 during the "priming" stage and Fig. 20 illustrating fluid flow within the separation chamber 54 during the "air harvest" stage. As best shown in Fig. 20, the separation chamber 54 includes an inlet port 196 and two outlet ports 198 and 200 (one being a "low-G" outlet port associated with line L3 of the fluid flow circuit 12 and the other being a "high-G" outlet port associated with line L4 of the fluid flow circuit 12). In the illustrated embodiment, the inlet port 196 and outlet ports 198 and 200 are positioned adjacent to each other (though one outlet port 200 is separated from the inlet port 196 by a terminal wall of the separation chamber 54), but it should be understood that, in other embodiments, the ports may be differently positioned about the central axis of the separation chamber 54 (e.g., with one port positioned 180° from the other two ports or with each port spaced 120° from the adjacent ports). Notably, the inlet port 196 is positioned at or adjacent to the bottom end 194 of the separation chamber 54 (which allows it to be positioned at a "low point" L of the chamber 54), while the outlet ports 196 and 198 are positioned at or adjacent to the upper end 192 of the separation chamber 54 (which allows them to be positioned at a "high point" H of the chamber 54).

When the separation chamber 54 is in the target orientation for a "priming" stage (as in Fig. 17), the outlet ports 198 and 200 are positioned at the "high point" H (Fig. 18). As shown in Fig. 18, this orientation causes the air "A" in the separation chamber 54 to move to the "high point" H, while priming fluid "P" within the separation chamber 54 moves toward the "low point" L. The angle θ of the centrifuge 22 and rotational axis R may be chosen to minimize the area to which air A will migrate towards in the chamber fluid path, which better directs the air A toward the outlet ports 198 and 200 at the "high point" H for air removal.

As explained above, during the "priming" stage, priming fluid P (which may be, for example, blood) is pumped into the separation chamber 54 via the inlet port 196 (which is associated with line L2 of the fluid flow circuit 12) to move air A out of the separation chamber 54 via the outlet ports 198 and 200 (and associated lines L3 and L4 of the fluid flow circuit 12). When the separation chamber 54 is positioned with the outlet ports 198 and 200 at the "high point" H of the chamber 54, priming fluid P will effectively fill the chamber 54 while forcing air A to the "high point" H and through the outlet ports 198 and 200, as depicted in the fluid path of Fig. 18.

In a variation of such a "priming" stage, rather than immediately rotating the separation chamber 54 into the orientation of Figs. 17 and 18, a volume of priming fluid P may first be pumped into the separation chamber 54 via the inlet port 196. With this initial volume of priming fluid P in the separation chamber 54, the controller may control the centrifuge 22 to rotate into an orientation that places the inlet port 196 at the "low point" L (as in Fig. 20), which forces the priming fluid P to fill the inlet port 196. Once the inlet port 196 has been filled with the priming fluid P, the controller may control the centrifuge 22 to rotate the separation chamber 54 into the target orientation of Figs. 17 and 18 for removal of air A from the chamber 54 via the outlet ports 198 and 200.

When the separation chamber 54 is in the target orientation for an "air harvest" stage (as in Fig. 19), the inlet port 196 is positioned at the "low point" L (Fig. 20). As shown in Fig. 20, this orientation causes any air A in the separation chamber 54 (including air A pumped into the separation chamber 54 during "air harvest" stage) to move toward the "high point" H, while the fluid and/or fluid components "F" remaining within the separation chamber 54 (which may be red blood cells, for example) moves toward the "low point" L. As explained above, during one version of the "air harvest" stage, air A is pumped into the separation chamber 54 via the "low-G" outlet port 198 (which is associated with line L3 of the fluid flow circuit 12) to move the fluid and/or fluid components F out of the separation chamber 54 via the inlet port 196 (and associated line L2 of the fluid flow circuit 12).

In a variation of the "air harvest" stage, fluid and/or fluid components F may be recovered from the chamber 54 via one or both of the outlet ports 198 and 200, rather than via the inlet port 196. This may include the pump system and the valve system being controlled so as to cause air to enter the chamber 54 via the inlet port 196 or via one of the outlet ports (with the air exiting the chamber 54 via the other outlet port). In such a variation, the controller may control the centrifuge 22 to rotate the separation chamber 54 into a target orientation that places whichever port is used for outflow of fluid and/or fluid components F into its lowest elevation (which is when that port is in angular alignment with the "low point" L). In the illustrated embodiment, the orientation of Fig. 20 is effective to place all three ports of the separation chamber 54 at their lowest point (such that any of them may be effectively used to recover fluid and/or fluid components F from the chamber 54 during an "air harvest" stage) but, in other embodiments in which the separation chamber 54 is differently configured, different orientations may be required to move the appropriate port into the "low point" L of the chamber 54 (or at least to its lowest point) for an "air harvest" stage.

It should be understood that the illustrated separation device 10, the fluid flow circuit 12, and the procedural stages are merely exemplary of a system and procedure that may employ the techniques and principles described herein. Indeed, differently configured fluid separation systems and separation procedures including different steps may employ the techniques and principles described herein without departing from the scope of the present disclosure.

### Aspects

Aspect 1. A blood processing device, comprising a pump system; a valve system; a separator; and a controller operably connected to the pump system, the valve system and the separator, the controller configured to execute a plasma collection stage of a blood processing procedure by: actuating the pump system and the valve system to convey blood from a blood source into the separator, actuating the separator to separate the blood in the centrifuge into at least plasma and red blood cells, in a transfusion plasma collection step of the plasma collection stage, actuating the pump system and the valve system to convey a first volume of the separated plasma out of the separator for collection, wherein the valve system is actuated to convey the first volume of the separated plasma along a first flow path for transfusion plasma collection, and in a fractionation plasma collection step of the plasma collection stage, actuating the pump system and valve system to convey a second volume of the separated plasma out of the centrifuge for collection, wherein the valve system is actuated to convey the second volume of the plasma along a second flow path for fractionation plasma collection.

Aspect 2. The blood processing device according to Aspect 1, wherein: the controller is further configured to determine the first volume of plasma conveyed out of the centrifuge and to execute the fractionation plasma collection stage when the first volume is equal to a target plasma volume for transfusion plasma collection.

Aspect 3. The blood processing device according to any one of the preceding Aspects, wherein the valve system comprises a first valve configured to control flow of the first volume of the separated plasma and a second valve configured to control flow of the second volume of the separated plasma, the first valve is open and the second valve is closed in the transfusion plasma collection step, and the first valve is closed and the second valve is open in the fractionation plasma collection step.

Aspect 4. The blood processing device according to any one of the preceding Aspects, wherein the pump system comprising a first pump and a second pump, and the controller is configured to actuate the first pump and the second pump during the plasma collection stage to simultaneously convey the blood into the separator and the separated plasma and the red blood cells out of the separator.

Aspect 5. The blood processing device according to Aspect 4, wherein the pump system further comprises a third pump, and the controller is configured to actuate the third pump to convey an additive solution from an additive solution supply to the separated red blood cells conveyed out of the separator to combine the separated red blood cells with the additive solution.

Aspect 6. A blood processing system, comprising: a reusable processing device including a pump system, a valve system a separator and a controller; and a disposable fluid flow circuit including a processing chamber interacting with the separator, a blood supply container, a transfusion plasma collection container, a fractionation plasma collection container and a red blood cell collection container, and a plurality of conduits connecting the processing chamber, the blood supply container, the transfusion plasma collection container, the fractionation plasma collection container and the red blood cell collection container, wherein the controller is configured to execute a plasma collection stage by: actuating the pump system and the valve system to convey blood from the blood supply container into the processing chamber, actuating the separator to separate the blood in the processing chamber into plasma and red blood cells, in a transfusion plasma collection step of the plasma collection stage, actuating the pump system and the valve system to convey a first volume of the separated plasma from the processing chamber via a first outlet for collection in the transfusion plasma collection container, wherein the valve system is actuated to convey the first volume of the separated plasma along a first flow path to the transfusion plasma collection container, in a fractionation plasma collection step, actuating the pump system and the valve system to convey a second volume of the separated plasma from the processing chamber via the first outlet for collection in the fractionation plasma collection container, wherein the valve system is actuated to convey the second volume of the separated plasma along a second flow path to the fractionation plasma collection container, and actuating the pump system and the valve system to convey the separated red blood cells from the processing chamber via a second outlet for collection in the red blood cell container.

Aspect 7. The blood processing system according to Aspect 6, wherein: in the transfusion plasma collection step, the controller is further configured to determine whether the first volume of the separated plasma corresponds to a target plasma volume for transfusion plasma collection, and in response to determining the first volume corresponds to the target plasma volume, the controller is further configured to end the transfusion plasma collection step and execute the fractionation plasma collection step.

Aspect 8. The blood processing system according to Aspect 7, wherein: in the fractionation plasma collection step, the controller is configured to determine whether a volume of blood in the blood supply container corresponds to a target blood volume, and in response to determining the volume of blood corresponds to the target blood volume, the controller is further configured to end the fractionation plasma collection step.

Aspect 9. The blood processing system according to any one of Aspects 6-8, wherein the valve system comprises a first valve configured to control flow of the first volume of the separated plasma and a second valve configured control flow of the second volume of separated plasma, the first valve is open and the second valve is closed in the transfusion plasma collection step, and the first valve is closed and the second valve is open in the fractionation plasma collection step.

Aspect 10. The blood processing system according to any one of Aspects 6-9, wherein the pump system comprises a first pump and a second pump, and the controller is configured to actuate the first pump and the second pump during the plasma collection stage to simultaneously convey blood from the blood supply container into the processing chamber and the separated plasma and separated red blood cells out of the processing chamber for collection.

Aspect 11. The blood processing system according to Aspect 10, wherein the pump system further comprises a third pump, and the controller is configured to actuate the third pump to convey an additive solution from an additive solution supply container to the separated red blood cells conveyed out of the processing chamber to combine the separated red blood cells with the additive solution.

Aspect 12. The blood processing system according to Aspect 6, wherein the separator comprises a centrifuge and the controller is further configured to execute a blood prime stage prior to the plasma collection stage by: actuating the pump system and the valve system to convey blood from the blood supply container into the processing chamber, actuating the centrifuge at a first rotation rate such that the blood remains unseparated, actuating the pump system and the valve system to convey a first portion of unseparated blood out of the processing chamber via the first outlet so as to convey air from the processing chamber to the transfusion plasma collection container via the first outlet along the first flow path, and actuating the pump system and the valve system to convey a second portion of the unseparated blood out of the processing chamber via the second outlet so as to convey air from the processing chamber to the transfusion plasma container via the second outlet along a third flow path.

Aspect 13. The blood processing system according to Aspect 12, wherein the controller is further configured to execute an establish separation stage after the blood prime stage and prior to the plasma collection stage by: actuating the pump system and the valve system to convey blood from the blood supply container into the processing chamber, actuating the centrifuge at a second rotation rate to separate the blood in the processing chamber into plasma and red blood cells, actuating the pump system and the valve system to convey the separated plasma from the processing chamber via the first outlet, actuating the pump system and the valve system to convey the separated red blood cells from the processing chamber via the second outlet, and actuating the pump system and the valve system to combine the separated plasma and the separated red blood cells as reconstituted blood and convey the reconstituted blood back into the processing chamber.

Aspect 14. The blood processing system according to Aspect 13, wherein the controller is further configured to execute a red blood cell recovery stage after the plasma collection stage by: actuating the pump system and the valve system to convey air from the transfusion plasma collection container into the processing chamber, actuating the centrifuge such that the air urges separated red blood cells in the processing chamber toward the second outlet, and actuating the pump system and the valve system to convey the separated red blood cells from the processing chamber to the red blood cell collection container.

Aspect 15. The blood processing system according to Aspect 14, wherein the fluid flow circuit further comprises an additive solution container connected to a fourth flow path extending between the processing chamber and the red blood cell collection container, and the controller is configured to further execute one or more of the plasma collection stage and the red blood cell recovery stage by actuating the pump system and the valve system to convey additive solution from the additive solution container to the fourth flow path to combine the additive solution with the separated red blood cells conveyed out of the processing chamber.

Aspect 16. The blood processing system according to Aspect 15, wherein the controller is further configured to execute an additive solution flush stage after the red blood cell recovery stage by actuating the pump system and the valve system to convey the additive solution from the additive solution container into the red blood cell collection container.

Aspect 17. The blood processing system according to Aspect 16, wherein the controller is further configured to execute an air evacuation stage after the additive solution flush stage by actuating the pump system and the valve system to draw air from the red blood cell collection container into the additive solution supply container.

Aspect 18. The blood processing system according to any one of Aspects 12-17, wherein the fluid flow circuit further comprises a leukoreduction filter arranged in a flow path between the second outlet and the red blood cell collection container, and the controller is configured to actuate the valve system to provide a bypass flow path around the leukoreduction filter.

Aspect 19. The blood processing system according to any one of Aspects 12-18, wherein the reusable processing device further comprises a transfusion plasma sensor configured to detect a transfusion plasma status and transmit transfusion plasma status information indicative of the detected transfusion plasma status to the controller, and a blood supply sensor configured to detect a blood supply status and transmit blood supply status information indicative of the detected blood supply status to the controller, in the transfusion plasma collection step of the plasma collection stage, the controller is configured to determine whether the first volume of the separated transfusion plasma corresponds to a target plasma volume based on the transfusion plasma status information, and in the fractionation plasma collection step of the plasma collection stage, the controller is configured to determine whether a volume of blood in the blood supply container corresponds to a target blood volume based on the blood supply status information.

Aspect 20. The blood processing system according to Aspect 19, wherein the detected transfusion plasma status is one or more of a weight of the transfusion plasma collection container and a volume of separated plasma moved by a pump of the pump system arranged between the processing chamber and the transfusion plasma collection container, and the detected blood supply status is one or more of a weight of the blood supply container and a hydrostatic pressure in a line downstream from the blood supply container.

Aspect 21. A fluid separation device, comprising: a pump system; a centrifuge configured to receive a separation chamber comprising an inlet port and an outlet port and to rotate the separation chamber about a non-vertical, non-horizontal rotational axis; and a controller programmed to execute a fluid separation procedure including a "priming" stage in which the controller controls the centrifuge to rotate the separation chamber to a first target orientation and then controls the pump system to convey a priming fluid into the separation chamber via the inlet port to remove air from the separation chamber via the outlet port while retaining the separation chamber in said first target orientation, and/or an "air harvest" stage in which the controller controls the centrifuge to rotate the separation chamber to a second target orientation and then controls the pump system to convey air into the separation chamber via one of the inlet port and the outlet port to remove fluid and/or a fluid component from the separation chamber via the other one of the inlet port and the outlet port while retaining the separation chamber in said second target orientation.

Aspect 22. The fluid separation device of Aspect 21, wherein the centrifuge is configured as a direct drive centrifuge.

Aspect 23. The fluid separation device of any one of Aspects 21-22, wherein the rotational axis is oriented at an angle in a range of approximately 30° to approximately 60° from horizontal.

Aspect 24. The fluid separation device of any one of Aspects 21-23, wherein the outlet port is positioned at a maximum elevation in the first target orientation.

Aspect 25. The fluid separation device of any one of Aspects 21-24, wherein air is conveyed into the separation chamber via the inlet port during the "air harvest" stage, and the outlet port is positioned at a minimum elevation in the second target orientation.

Aspect 26. The fluid separation device of any one of Aspects 21-24, wherein air is conveyed into the separation chamber via the outlet port during the "air harvest" stage, and the inlet port is positioned at a minimum elevation in the second target orientation.

Aspect 27. The fluid separation device of any one of Aspects 21-26, wherein the centrifuge includes a receptacle configured to be secured to the separation chamber via a keyed connection, the centrifuge includes a motor, and the controller is programmed to control the motor to move to a first motor position to place the separation chamber in the first target orientation and/or to control the motor to move to a second motor position to place the separation chamber in the second target orientation.

Aspect 28. The fluid separation device of any one of Aspects 21-27, further comprising an optical system configured to transmit signals to the controller that are indicative of an orientation of the separation chamber, wherein the controller is programmed to control rotation of the centrifuge so as to place the separation chamber in the first target orientation and/or the second target orientation based at least in part on said signals.

Aspect 29. A method of priming a separation chamber received within a centrifuge, comprising: controlling the centrifuge to rotate the separation chamber about a non-vertical, non-horizontal rotational axis to a target orientation; and controlling a pump system to convey a priming fluid into the separation chamber via an inlet port to remove air from the separation chamber via an outlet port while retaining the separation chamber in said target orientation.

Aspect 30. The method of Aspect 29, wherein the centrifuge is rotated via a direct drive assembly.

Aspect 31. The method of any one of Aspects 29-30, wherein the rotational axis is oriented at an angle in a range of approximately 30° to approximately 60° from horizontal.

Aspect 32. The method of any one of Aspects 29-31, wherein the outlet port is positioned at a maximum elevation in the target orientation.

Aspect 33. The method of any one of Aspects 29-32, wherein the separation chamber is secured to the centrifuge via a keyed connection, and rotating the separation chamber to the target orientation includes controlling a motor of the centrifuge to move to a target motor position so as to place the separation chamber in the target orientation.

Aspect 34. The method of any one of Aspects 29-33, wherein rotating the separation chamber to the target orientation includes optically detecting an orientation of the separation chamber.

Aspect 35. A method of harvesting a fluid and/or a fluid component from a separation chamber received within a centrifuge, comprising: controlling the centrifuge to rotate the separation chamber about a non-vertical, non-horizontal rotational axis to a target orientation; and controlling a pump system to convey air into the separation chamber via a first port to remove a fluid and/or a fluid component from the separation chamber via a second port while retaining the separation chamber in said target orientation.

Aspect 36. The method of Aspect 35, wherein the centrifuge is rotated via a direct drive assembly.

Aspect 37. The method of any one of Aspects 35-36, wherein the rotational axis is oriented at an angle in a range of approximately 30° to approximately 60° from horizontal.

Aspect 38. The method of any one of Aspects 35-37, wherein the second port is positioned at a minimum elevation in the target orientation.

Aspect 39. The method of any one of Aspects 35-38, wherein the separation chamber is secured to the centrifuge via a keyed connection, and rotating the separation chamber to the target orientation includes controlling a motor of the centrifuge to move to a target motor position so as to place the separation chamber in the target orientation.

Aspect 40. The method of any one of Aspects 35-39, wherein rotating the separation chamber to the target orientation includes optically detecting an orientation of the separation chamber.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood processing device, comprising:
a pump system;
a valve system;
a separator; and
a controller operably connected to the pump system, the valve system and the separator, the controller configured to execute a plasma collection stage of a blood processing procedure by:
actuating the pump system and the valve system to convey blood from a blood source into the separator,
actuating the separator to separate the blood in the separator into at least plasma and red blood cells,
in a transfusion plasma collection step of the plasma collection stage, actuating the pump system and the valve system to convey a first volume of the separated plasma out of the separator for collection, wherein the valve system is actuated to convey the first volume of the separated plasma along a first flow path for transfusion plasma collection, and
in a fractionation plasma collection step of the plasma collection stage, actuating the pump system and valve system to convey a second volume of the separated plasma out of the separator for collection, wherein the valve system is actuated to convey the second volume of the plasma along a second flow path for fractionation plasma collection.

2. The blood processing device according to claim 1, wherein the controller is further configured to determine the first volume of plasma conveyed out of the separator and to execute the fractionation plasma collection stage when the first volume is equal to a target plasma volume for transfusion plasma collection.

3. The blood processing device according to any one of the preceding claims, wherein
the valve system comprises a first valve configured to control flow of the first volume of the separated plasma and a second valve configured to control flow of the second volume of the separated plasma,
the first valve is open and the second valve is closed in the transfusion plasma collection step, and
the first valve is closed and the second valve is open in the fractionation plasma collection step.

4. The blood processing device according to any one of the preceding claims, wherein
the pump system comprises a first pump and a second pump, and
the controller is configured to actuate the first pump and the second pump during the plasma collection stage to simultaneously convey the blood into the separator and the separated plasma and the red blood cells out of the separator.

5. The blood processing device according to claim 4, wherein
the pump system further comprises a third pump, and
the controller is configured to actuate the third pump to convey an additive solution from an additive solution supply to the separated red blood cells conveyed out of the separator to combine the separated red blood cells with the additive solution.

6. The blood processing device according to claim 1, wherein
the separator comprises a centrifuge configured to receive a separation chamber comprising an inlet port and an outlet port and to rotate the separation chamber about a non-vertical, non-horizontal rotational axis, and
the controller is further programmed to execute a fluid separation procedure including
a "priming" stage in which the controller controls the centrifuge to rotate the separation chamber to a first target orientation and then controls the pump system to convey a priming fluid into the separation chamber via the inlet port to remove air from the separation chamber via the outlet port while retaining the separation chamber in said first target orientation, and/or
an "air harvest" stage in which the controller controls the centrifuge to rotate the separation chamber to a second target orientation and then controls the pump system to convey air into the separation chamber via one of the inlet port and the outlet port to remove fluid and/or a fluid component from the separation chamber via the other one of the inlet port and the outlet port while retaining the separation chamber in said second target orientation.

7. A blood processing system, comprising:
the processing device of any one of the preceding claims; and
a disposable fluid flow circuit including a processing chamber interacting with the separator, a blood supply container, a transfusion plasma collection container, a fractionation plasma collection container and a red blood cell collection container, and a plurality of conduits connecting the processing chamber, the blood supply container, the transfusion plasma collection container, the fractionation plasma collection container and the red blood cell collection container, wherein the controller is configured to execute a plasma collection stage by:
actuating the pump system and the valve system to convey blood from the blood supply container into the processing chamber,
actuating the separator to separate the blood in the processing chamber into plasma and red blood cells,
in the transfusion plasma collection step of the plasma collection stage, actuating the pump system and the valve system to convey a first volume of the separated plasma from the processing chamber via a first outlet for collection in the transfusion plasma collection container, wherein the valve system is actuated to convey the first volume of the separated plasma along a first flow path to the transfusion plasma collection container,
in a fractionation plasma collection step, actuating the pump system and the valve system to convey a second volume of the separated plasma from the processing chamber via the first outlet for collection in the fractionation plasma collection container, wherein the valve system is actuated to convey the second volume of the separated plasma along a second flow path to the fractionation plasma collection container, and
actuating the pump system and the valve system to convey the separated red blood cells from the processing chamber via a second outlet for collection in the red blood cell container.

8. The blood processing system according to claim 7, wherein:
in the fractionation plasma collection step, the controller is configured to determine whether a volume of blood in the blood supply container corresponds to a target blood volume, and
in response to determining the volume of blood corresponds to the target blood volume, the controller is further configured to end the fractionation plasma collection step.

9. The blood processing system according to claim 7, wherein the separator comprises a centrifuge and the controller is further configured to execute a blood prime stage prior to the plasma collection stage by:
actuating the pump system and the valve system to convey blood from the blood supply container into the processing chamber,
actuating the centrifuge at a first rotation rate such that the blood remains unseparated,
actuating the pump system and the valve system to convey a first portion of unseparated blood out of the processing chamber via the first outlet so as to convey air from the processing chamber to the transfusion plasma collection container via the first outlet along the first flow path, and
actuating the pump system and the valve system to convey a second portion of the unseparated blood out of the processing chamber via the second outlet so as to convey air from the processing chamber to the transfusion plasma container via the second outlet along a third flow path.

10. The blood processing system according to claim 9, wherein the controller is further configured to execute an establish separation stage after the blood prime stage and prior to the plasma collection stage by:
actuating the pump system and the valve system to convey blood from the blood supply container into the processing chamber,
actuating the centrifuge at a second rotation rate to separate the blood in the processing chamber into plasma and red blood cells,
actuating the pump system and the valve system to convey the separated plasma from the processing chamber via the first outlet,
actuating the pump system and the valve system to convey the separated red blood cells from the processing chamber via the second outlet, and
actuating the pump system and the valve system to combine the separated plasma and the separated red blood cells as reconstituted blood and convey the reconstituted blood back into the processing chamber.

11. The blood processing system according to claim 10, wherein the controller is further configured to execute a red blood cell recovery stage after the plasma collection stage by:
actuating the pump system and the valve system to convey air from the transfusion plasma collection container into the processing chamber,
actuating the centrifuge such that the air urges separated red blood cells in the processing chamber toward the second outlet, and
actuating the pump system and the valve system to convey the separated red blood cells from the processing chamber to the red blood cell collection container.

12. The blood processing system according to claim 11, wherein
the fluid flow circuit further comprises an additive solution container connected to a fourth flow path extending between the processing chamber and the red blood cell collection container, and
the controller is configured to further execute one or more of the plasma collection stage and the red blood cell recovery stage by actuating the pump system and the valve system to convey additive solution from the additive solution container to the fourth flow path to combine the additive solution with the separated red blood cells conveyed out of the processing chamber.

13. The blood processing system according to claim 12, wherein the controller is further configured to execute at least one of
an additive solution flush stage by actuating the pump system and the valve system to convey the additive solution from the additive solution container into the red blood cell collection container, and
an air evacuation stage by actuating the pump system and the valve system to draw air from the red blood cell collection container into the additive solution supply container.

14. The blood processing system according to any one of claims 9-13, wherein
the processing device further comprises
a transfusion plasma sensor configured to detect a transfusion plasma status and transmit transfusion plasma status information indicative of the detected transfusion plasma status to the controller, and
a blood supply sensor configured to detect a blood supply status and transmit blood supply status information indicative of the detected blood supply status to the controller,
in the transfusion plasma collection step of the plasma collection stage, the controller is configured to determine whether the first volume of the separated transfusion plasma corresponds to a target plasma volume based on the transfusion plasma status information, and
in the fractionation plasma collection step of the plasma collection stage, the controller is configured to determine whether a volume of blood in the blood supply container corresponds to a target blood volume on the blood supply status information.

15. The blood processing system according to claim 14, wherein
the detected transfusion plasma status is one or more of a weight of the transfusion plasma collection container and a volume of separated plasma moved by a pump of the pump system arranged between the processing chamber and the transfusion plasma collection container, and
the detected blood supply status is one or more of a weight of the blood supply container and a hydrostatic pressure in a line downstream from the blood supply container.
